(19) Europäisches Patentamt / European Patent Office / Office européen des brevets

(11) **EP 2 162 550 B1**

(12) **EUROPEAN PATENT SPECIFICATION**

(45) Date of publication and mention of the grant of the patent:
**09.08.2017 Bulletin 2017/32**

(21) Application number: **08761038.2**

(22) Date of filing: **13.06.2008**

(51) Int Cl.:
*C12Q 1/68* *(2006.01)*

(86) International application number:
**PCT/EP2008/057519**

(87) International publication number:
**WO 2008/152146 (18.12.2008 Gazette 2008/51)**

(54) **PERIPHERICAL TISSUE SAMPLE CONTAINING CELLS EXPRESSING THE 5HTR2C AND/OR ADARS AS MARKERS OF THE ALTERATION OF THE MECHANISM OF THE 5HTR2C MRNA EDITING AND ITS APPLICATIONS**

PERIPHERE GEWEBEPROBE MIT 5HTR2C UND/ODER ADAR EXPRIMIERENDEN ZELLEN ALS MARKER FÜR DIE ÄNDERUNG DES MECHANISMUS DER 5HTR2C-MRNA-PROZESSIERUNG SOWIE ANWENDUNGEN DAVON

ECHANTILLON DE TISSU PERIPHERIQUE CONTENANT DES CELLULES EXPRIMANT 5HTR2C ET/OU LES ADAR, UTILISEES COMME MARQUEURS DE L'ALTERATION DU MECANISME D'EDITION DE L'ARNM 5HTR2C, ET SES APPLICATIONS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.06.2007 US 943685 P**
**24.01.2008 US 23239 P**

(43) Date of publication of application:
**17.03.2010 Bulletin 2010/11**

(73) Proprietor: **ALCEDIAG**
**13790 Peynier (FR)**

(72) Inventors:
• **WEISSMANN, Dinah**
**F-75005 Paris (FR)**
• **PUJOL, Jean-François**
**F-75005 Paris (FR)**
• **VINCENT, Laurent**
**F-91620 La ville du bois France (FR)**
• **CAVAREC, Laurent**
**F-94300 Vincennes (FR)**
• **MANN, John**
**Bronx, New York 10471 (US)**

(74) Representative: **Touroude, Magali Linda**
**Touroude & Associates**
**1, rue Albert Einstein**
**Champs sur Marne**
**77436 Marne la vallée Cedex (FR)**

(56) References cited:
**WO-A-2004/011594    WO-A-2005/087949**
**WO-A-2006/032859    WO-A-2006/134128**
**US-A- 5 763 174**

• **DATABASE DBSNP ncbi; 8 March 2007 (2007-03-08), "rs46360913" XP002498424 retrieved from HTTP://WWW.NCBI.NLM.NIH.GOV/SNP/SNP_REF.CG I?RS=46360913 Database accession no. rs46360913**
• **YANG WEIDONG ET AL: "Altered RNA editing of serotonin 5-HT2C receptor induced by interferon: implications for depression associated with cytokine therapy." BRAIN RESEARCH. MOLECULAR BRAIN RESEARCH 29 APR 2004, vol. 124, no. 1, 29 April 2004 (2004-04-29), pages 70-78, XP002498423 ISSN: 0169-328X cited in the application**

- OLGA A SERGEEVA ET AL: "Editing of AMPA and Serotonin 2C Receptors in Individual Central Neurons, Controlling Wakefulness" CELLULAR AND MOLECULAR NEUROBIOLOGY, KLUWER ACADEMIC PUBLISHERS-PLENUM PUBLISHERS, NE, vol. 27, no. 5, 7 June 2007 (2007-06-07), pages 669-680, XP019524896 ISSN: 1573-6830 cited in the application
- MARAZZITI D ET AL: "mRNA expression of serotonin receptors of type 2C and 5A in human resting lymphocytes" NEUROPSYCHOBIOLOGY, KARGER AG, BASEL, CH, vol. 43, no. 3, 1 January 2001 (2001-01-01), pages 123-126, XP008096962 ISSN: 0302-282X
- BARBON A ET AL: "Glutamate receptor RNA editing: A molecular analysis of GluR2, GluR5 and GluR6 in human brain tissues and in NT2 cells following in vitro neural differentiation" MOLECULAR BRAIN RESEARCH, ELSEVIER SCIENCE BV, AMSTERDAM, NL, vol. 117, no. 2, 1 October 2003 (2003-10-01), pages 168-178, XP002355586 ISSN: 0169-328X
- SCHMAUSS C: "SEROTONIN 2C RECEPTORS: SUICIDE, SEROTONIN, AND RUANWAY RNA EDITING" NEUROSCIENTIST, BALTIMORE, MD, US, vol. 9, no. 4, 1 August 2003 (2003-08-01), pages 237-242, XP009033018 ISSN: 1073-8584
- SHIN KWAK ET AL: "Deficient RNA editing of GluR2 and neuronal death in amyotropic lateral sclerosis" JOURNAL OF MOLECULAR MEDICINE, SPRINGER, BERLIN, DE, vol. 83, no. 2, 1 February 2005 (2005-02-01), pages 110-120, XP019320322 ISSN: 1432-1440
- NISWENDER COLLEEN M ET AL: "RNA editing of the human serotonin 5-HT2C receptor: Alterations in suicide and implications for serotonergic pharmacotherapy" NEUROPSYCHOPHARMACOLOGY, ELSEVIER SCIENCE PUBLISHING, NEW YORK, NY, US, vol. 24, no. 5, 1 May 2001 (2001-05-01), pages 478-491, XP002288022 ISSN: 0893-133X cited in the application
- SODHI M S ET AL: "RNA EDITING OF THE 5-HT2C RECEPTOR IS REDUCED IN SCHIZOPHRENIA" MOLECULAR PSYCHIATRY, BASINGSTOKE, GB, vol. 6, 1 January 2001 (2001-01-01), pages 373-379, XP008040842 ISSN: 1359-4184 cited in the application
- Andrzej Slominski ET AL: "Functional activity of serotoninergic and melatoninergic systems expressed in the skin", Journal of Cellular Physiology, vol. 196, no. 1, 20 May 2003 (2003-05-20), pages 144-153, XP055048966, ISSN: 0021-9541, DOI: 10.1002/jcp.10287
- GAN Z. ET AL: 'RNA Editing by ADAR2 Is Metabolically Regulated in Pancreatic Islets and beta-Cells' JOURNAL OF BIOLOGICAL CHEMISTRY vol. 281, no. 44, 06 September 2006, pages 33386 - 33394, XP055048994 DOI: 10.1074/jbc.M604484200 ISSN: 0021-9258
- CHARLES M.A. ET AL: 'Build an algorithm based on the following Development of an Array-Comparative Genomic Hybridization (aCGH)-Based Algorithm to Assist Renal Tumor Subtyping in Needle Biopsies and Resected Specimens Departments of b Surgery, c Interventional Radiology and Image Guided Therapies, d Medicine, and e Path' INTERNET ARTICLE, [Online] 13 February 2013, XP055112982 Retrieved from the Internet: <URL:http://www.cgifhact.com/wp-content/uploads/2013/12/AMP-2013-poster-UroGenRA.pdf> [retrieved on 2014-04-09]

SEQUENCE LISTING

<110> BIOCORTECH
WEISSMANN, Dinah
PUJOL, Jean-François
VINCENT, Laurent
CAVAREC, Laurent
MANN, John

<120> Peripherical tissue sample containing cells expressing the 5HTR2C and/or ADARs as markers of the alteration of the mechanism of the 5HTR2C mRNA editing and its applications

<130> D25626

<150> 60/943685
<151> 2007-06-13

<150> 61/023239
<151> 2008-01-24

<160> 51

<170> PatentIn version 3.3

<210> 1
<211> 15
<212> DNA
<213> Artificial

<220>
<223> Description of the artificial sequence : DNA derived from the mRNA coding for 5-HT2C receptor

<400> 1
atacgtaatc ctatt          15

<210> 2
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<400> 2
ntacgtaatc ctatt          15

<210> 3
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<400> 3
atncgtaatc ctatt          15

<210> 4
<211> 15
<212> DNA
<213> Artificial

<220>
<223> Description of the artificial sequence : DNA derived from the mRNA coding for 5-HT2C receptor

<220>
<221> modified_base
<222> (8)..(8)
<223> n=i

<400> 4
atacgtantc ctatt          15

<210> 5
<211> 15
<212> DNA
<213> Artificial

<220>
<223> Description of the artificial sequence : DNA derived from the mRNA coding for 5-HT2C receptor

<220>
<221> modified_base
<222> (13)..(13)
<223> n=i

<400> 5
atacgtaatc ctntt          15

<210> 6
<211> 15
<212> DNA
<213> Artificial

<220>
<223> Description of the artificial sequence : DNA derived from the mRNA coding for 5-HT2C receptor

<220>
<221> modified_base
<222> (7)..(7)
<223> n=i

<400> 6
atacgtnatc ctatt          15

<210> 7
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (3)..(3)
<223> n=i

<400> 7
ntncgtaatc ctatt          15

<210> 8
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (8)..(8)
<223> n=i

<400> 8
ntacgtantc ctatt          15

<210> 9
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (13)..(13)

<223> n=i

<400> 9
ntacgtaatc ctntt        15

<210> 10
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (7)..(7)
<223> n=i

<400> 10
ntacgtnatc ctatt        15

<210> 11
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base
<222> (8)..(8)
<223> n=i

<400> 11
atncgtantc ctatt        15

<210> 12
<211> 15
<212> DNA
<213> Artificial

<220>
<223> Description of the artificial sequence : DNA derived from the mRNA coding for 5-HT2C receptor

<220>
<221> modified_base
<222> (3)..(3)

<223> n=i

<220>
<221> modified_base
<222> (13)..(13)
<223> n=i

<400> 12
atncgtaatc ctntt          15

<210> 13
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base
<222> (7)..(7)
<223> n=i

<400> 13
atncgtnatc ctatt          15

<210> 14
<211> 15
<212> DNA
<213> Artificial

<220>
<223> Description of the artificial sequence : DNA derived from the mRNA coding for 5-HT2C receptor

<220>
<221> modified_base
<222> (8)..(8)
<223> n=i

<220>
<221> modified_base
<222> (13)..(13)
<223> n=i

<400> 14
atacgtantc ctntt          15

<210> 15
<211> 15
<212> DNA
<213> Artificial

<220>

<223> Description of the artificial sequence : DNA derived from the mRNA coding for 5-HT2C receptor

<220>
<221> modified_base
<222> (7)..(7)
<223> n=i

<220>
<221> modified_base
<222> (8)..(8)
<223> n=i

<400> 15
atacgtnntc ctatt          15

<210> 16
<211> 15
<212> DNA
<213> Artificial

<220>
<223> Description of the artificial sequence : DNA derived from the mRNA coding for 5-HT2C receptor

<220>
<221> modified_base
<222> (7)..(7)
<223> n=i

<220>
<221> modified_base
<222> (13)..(13)
<223> n=i

<400> 16
atacgtnatc ctntt          15

<210> 17
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (3)..(3)
<223> n=i

<220>
<221> modified_base
<222> (8)..(8)
<223> n=i

<400> 17
ntncgtantc ctatt          15


<210> 18
<211> 15
<212> DNA
<213> Artificial


<220>
<223> Description of the artificial sequence : DNA derived from the mRNA coding for 5-HT2C receptor


<220>
<221> modified_base
<222> (1)..(1)
<223> n=i


<220>
<221> modified_base
<222> (3)..(3)
<223> n=i


<220>
<221> modified_base
<222> (13)..(13)
<223> n=i


<400> 18
ntncgtaatc ctntt          15


<210> 19
<211> 15
<212> DNA
<213> Artificial


<220>
<223> Description of the artificial sequence : DNA derived from the mRNA coding for 5-HT2C receptor


<220>
<221> modified_base
<222> (1)..(1)
<223> n=i


<220>
<221> modified_base
<222> (3)..(3)
<223> n=i


<220>
<221> modified_base
<222> (7)..(7)
<223> n=i


<400> 19
ntncgtnatc ctatt          15


<210> 20
<211> 15
<212> DNA

<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (8)..(8)
<223> n=i

<220>
<221> modified_base
<222> (13)..(13)
<223> n=i

<400> 20
ntacgtantc ctntt          15

<210> 21
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (7)..(7)
<223> n=i

<220>
<221> modified_base
<222> (8)..(8)
<223> n=i

<400> 21
ntacgtnntc ctatt          15

<210> 22
<211> 15
<212> DNA
<213> Artificial

<220>
<223> Description of the artificial sequence : DNA derived from the mRNA coding for 5-HT2C receptor

<220>

<221> modified_base
<222> (1)..(1)
<223> n=i

<220>
<221> modified_base
<222> (7)..(7)
<223> n=i

<220>
<221> modified_base
<222> (13)..(13)
<223> n=i

<400> 22
ntacgtnatc ctntt          15

<210> 23
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base
<222> (8)..(8)
<223> n=i

<220>
<221> modified_base
<222> (13)..(13)
<223> n=i

<400> 23
atncgtantc ctntt          15

<210> 24
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base

<222> (7)..(7)
<223> n=i

<220>
<221> modified_base
<222> (8)..(8)
<223> n=i

<400> 24
atncgtnntc ctatt        15

<210> 25
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base
<222> (7)..(7)
<223> n=i

<220>
<221> modified_base
<222> (13)..(13)
<223> n=i

<400> 25
atncgtnatc ctntt        15

<210> 26
<211> 15
<212> DNA
<213> Artificial

<220>
<223> Description of the artificial sequence : DNA derived from the mRNA coding for 5-HT2C receptor

<220>
<221> modified_base
<222> (7)..(7)
<223> n=i

<220>
<221> modified_base
<222> (8)..(8)
<223> n=i

<220>
<221> modified_base
<222> (13)..(13)

<223> n=i

<400> 26
atacgtnntc ctntt        15

<210> 27
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (3)..(3)
<223> n=i

<220>
<221> modified_base
<222> (8)..(8)
<223> n=i

<220>
<221> modified_base
<222> (13)..(13)
<223> n=i

<400> 27
ntncgtantc ctntt        15

<210> 28
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (3)..(3)
<223> n=i

<220>
<221> modified_base
<222> (7)..(7)
<223> n=i

<220>
<221> modified_base
<222> (8)..(8)
<223> n=i

<400> 28
ntncgtnntc ctatt          15

<210> 29
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (3)..(3)
<223> n=i

<220>
<221> modified_base
<222> (7)..(7)
<223> n=i

<220>
<221> modified_base
<222> (13)..(13)
<223> n=i

<400> 29
ntncgtnatc ctntt          15

<210> 30
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base
<222> (7)..(7)
<223> n=i

<220>

<221> modified_base
<222> (8)..(8)
<223> n=i

<220>
<221> modified_base
<222> (13)..(13)
<223> n=i

<400> 30
ntacgtnntc ctntt           15

<210> 31
<211> 15
<212> DNA
<213> Artificial

<220>
<223> Description of the artificial sequence : DNA derived from the mRNA coding for 5-HT2C receptor

<220>
<221> modified_base
<222> (3)..(3)
<223> n=i

<220>
<221> modified_base
<222> (7)..(7)
<223> n=i

<220>
<221> modified_base
<222> (8)..(8)
<223> n=i

<220>
<221> modified_base
<222> (13)..(13)
<223> n=i

<400> 31
atncgtnntc ctntt           15

<210> 32
<211> 15
<212> DNA
<213> Artificial

<220>
<223> Description of the artificial sequence : DNA derived from the mRNA coding for 5-HT2C receptor

<220>
<221> modified_base
<222> (1)..(1)
<223> n=i

<220>
<221> modified_base

<222> (3)..(3)
<223> n=i

<220>
<221> modified_base
<222> (7)..(7)
<223> n=i

<220>
<221> modified_base
<222> (8)..(8)
<223> n=i

<220>
<221> modified_base
<222> (13)..(13)
<223> n=i

<400> 32
ntncgtnntc ctntt            15

<210> 33
<211> 15
<212> RNA
<213> Artificial

<220>
<223> Description of the artificial sequence : sequence derived from the cDNA coding for 5-HT2C receptor

<400> 33
auacguaauc cuauu            15

<210> 34
<211> 21
<212> DNA
<213> Artificial

<220>
<223> Description of the artificial sequence : sequence derived from the cDNA coding for 5-HT2C receptor

<400> 34
ttcgtccctc agtccaatca c            21

<210> 35
<211> 24
<212> DNA
<213> Artificial

<220>
<223> Description of the artificial sequence : sequence derived from the cDNA coding for 5-HT2C receptor

<400> 35
tgtccctagc cattgctgat atgc            24

<210> 36
<211> 24
<212> DNA
<213> Artificial

<220>
<223> Description of the artificial sequence : sequence derived from the cDNA coding for 5-HT2C receptor

<400> 36
gcaatcttca tgatggcctt agtc          24

<210> 37
<211> 26
<212> DNA
<213> Artificial

<220>
<223> Description of the artificial sequence : sequence derived from the cDNA coding for 5-HT2C receptor

<400> 37
atgtgctatt ttcaacagcg tccatc          26

<210> 38
<211> 21
<212> DNA
<213> Artificial

<220>
<223> Description of the artificial sequence : sequence derived from the cDNA coding for 5-HT2C receptor

<400> 38
gcaatcttca tgatggcctt a          21

<210> 39
<211> 18
<212> DNA
<213> Artificial

<220>
<223> Description of the artificial sequence : sequence derived from the cDNA coding for 5-HT2C receptor

<400> 39
tttgtgcccc gtctggat          18

<210> 40
<211> 18
<212> DNA
<213> Artificial

<220>
<223> Description of the artificial sequence : sequence derived from the cDNA coding for 5-HT2C receptor

<400> 40
gccttagtcc gcgaattg          18

<210> 41
<211> 22
<212> DNA
<213> Artificial

<220>
<223> Description of the artificial sequence : sequence derived from the cDNA coding for human ADAR1 150 isoform

<400> 41
gcctcgcggg cgcaatgaat cc            22


<210> 42
<211> 22
<212> DNA
<213> Artificial


<220>
<223> Description of the artificial sequence : sequence derived from the cDNA coding for human ADAR1 150 isoform


<400> 42
cttgcccttc tttgccaggg ag            22


<210> 43
<211> 24
<212> DNA
<213> Artificial


<220>
<223> Description of the artificial sequence : sequence derived from the cDNA coding for human ADAR1 110 isoform


<400> 43
cgagccatca tggagatgcc ctcc            24


<210> 44
<211> 24
<212> DNA
<213> Artificial


<220>
<223> Description of the artificial sequence : sequence derived from the cDNA coding for human ADAR1 110 isoform


<400> 44
catagctgca tcctgcttgg ccac            24


<210> 45
<211> 23
<212> DNA
<213> Artificial


<220>
<223> Description of the artificial sequence : sequence derived from the cDNA coding for human ADAR2


<400> 45
gctgcgcagt ctgccctggc cgc            23


<210> 46
<211> 23
<212> DNA
<213> Artificial


<220>
<223> Description of the artificial sequence : sequence derived from the cDNA coding for human ADAR2


<400> 46
gtcatgacga ctccagccag cac            23

<210> 47
<211> 22
<212> DNA
<213> Artificial

<220>
<223> Description of the artificial sequence : sequence derived from the cDNA coding for murine ADAR1 150 isoform

<400> 47
gtctcaaggg ttcaggggac cc          22

<210> 48
<211> 24
<212> DNA
<213> Artificial

<220>
<223> Description of the artificial sequence : sequence derived from the cDNA coding for murine ADAR1 150 isoform

<400> 48
ctcctctagg gaattcctgg atac          24

<210> 49
<211> 23
<212> DNA
<213> Artificial

<220>
<223> Description of the artificial sequence : sequence derived from the cDNA coding for murine ADAR1 110 isoform

<400> 49
tcacgagtgg gcagcgtccg agg          23

<210> 50
<211> 23
<212> DNA
<213> Artificial

<220>
<223> Description of the artificial sequence : sequence derived from the cDNA coding for murine ADAR2

<400> 50
gctgcacagt ctgccttggc tac          23

<210> 51
<211> 24
<212> DNA
<213> Artificial

<220>
<223> Description of the artificial sequence : sequence derived from the cDNA coding for murine ADAR2

<400> 51
gcataaagaa acctgagcag ggac          24

**Description**

The present invention relates to an *in vitro* method for predicting a pathology related to an alteration of the mechanism of the mRNA editing of ADAR dependent A to I mRNA editing, particularly the serotonin 2C receptor (5HTR2C), in a patient from a peripherical tissue sample containing cells expressing said mRNA, such as the 5HTR2C mRNA, and/or adenosine deaminases acting on RNA (ADARs), such as skin tissue sample. The present invention further comprises the use of a method according to the invention for identifying if an agent is capable of in vivo modifying the editing of the 5HTR2C mRNA in brain tissue or to control the efficiency of a drug intended to prevent or to treat a pathology related to an alteration of the mechanism of the 5HTR2C mRNA editing brain tissue, these methods comprising the implementation of said peripherical tissue markers. In a particular aspect, the present invention relates to such methods wherein the 5HTR2C mRNA editing rate or profile, when it is necessary, is determined by a single strand conformation polymorphism (SSCP) method after amplification by a PCR, preferably by a nested PCR, of the specific mRNA fragment containing the edition sites, making it possible, under given analytical conditions, to obtain the editing rate and/or profile of this edited 5HTR2C mRNA from said peripherical tissue. Finally the invention relates to particular nucleic acid primers implemented in said nested PCR.

Genetic association studies, knockout mice and postmortem analysis have suggested the implication of the serotonin 2C receptor (5HTR2C) in neuropsychiatric disorders. Firstly, a functional allelic polymorphism (Cys23Ser) of 5HTR2C is associated with depression and bipolar disorder (Lerer et al., 2001, Mol. Psychiatry, 6:579-585). Secondly, mice lacking the 5HT2C serotonin receptor exhibit spontaneous convulsions, cognitive impairment and abnormal control of feeding behavior (Tecott et al., 1995, Nature, 374:542-546). These animals are also hyper responsive to repeated stress (Chou-green et al., 2003, Physiol. Behav., 79:217-226). Thirdly, in postmortem brains of patients affected by bipolar disorder or schizophrenia, the RNA expression of the 5-HT2C serotonin receptor is down-regulated (Iwamoto et al., 2004, Mol. Psychiatry, 9:406-416; Castensson et al., 2003, Biol. Psychiatry, 54:1212-1221).

RNA editing of 5HTR2C is also thought to be involved in the pathophysiology of mental disorders and the action of antidepressants (Seeburg, 2002, Neuron, 35:17-20). Five adenosine residues are edited in a region coding for the second intracellular loop of the 5HTR2C and can lead to amino-acid substitutions at three different positions of the receptor sequence. The combinational substitution of these amino residues generates up to 24 different 5HTR2C protein isoforms with different G-coupling efficiencies (Price et al., 2001, J. Biol. Chem., 276:44663-44668). In mice, when compared with C57BL/6 and 129sv inbred strains, the BALB/c strain exhibits a different basal forebrain neocortical 5HTR2C pre-mRNA editing pattern that may underlie their difference in stress reactivity. Moreover, the BALB/c mice exhibit stress-induced changes in 5HTR2C pre-mRNA editing resembling those detected in brains of depressed suicide victims (Englander et al., 2005, J. Neurosci., 25:648-651). Actually, in postmortem brains, altered RNA editing of 5HTR2C has been reported in patients with schizophrenia, depression and those who committed suicide (Niswender et al., 2001, Neuropsychopharmacology, 24:478-491; Sodhi et al., 2001, Mol. Psychiatry, 6:373-379; Gurevich et al., 2002, Neuron, 34:349-356). Additionally interferon is used in hepatitis C treatment but symptoms of depression often appear as a side effect of this molecule in patients and Yang et al. have demonstrated that this molecule strongly alters the editing of 5HTR2C (see ref. in Tohda et al., 2006, J. Pharmacol. Sci., 100, 427-432).

Previous studies have shown that the 5HTR2C is mainly expressed in the brain, particularly in choroid plexus, prefrontal cortex, limbic areas, basal ganglia and hypothalamus (Tohda et al., 1996, Neurosci. Res., 24:189-193; Julius et al., 1988, 241:558-564; Pasqualetti et al., 1999, Neuroscience, 92:601-611). This brain specific pattern of expression restricts investigations of potential links existing between 5HTR2C RNA editing and psychiatric condition to post-mortem studies. In search of more easily available tissues, possibly mirroring the editing status of HTR2C in CNS, and allowing quantitative analysis in patients with different psychiatric states, Marazziti and collaborators have detected the presence of 5HTR2C mRNAs in resting lymphocytes (Marazziti et al., 2001, Neuropsychobiology, 43:123-126). Unfortunately in these cells the level of expression of the 5HTR2C, as revealed by RT-PCR / Southern-blotting, is too low for further quantitative RNA editing analysis.

Recently, Slominski and co-workers have shown that human skin and cultured skin-derived cells have the capability to transform L-tryptophan to serotonin and to metabolize serotonin to N-acetyl-serotonin and melatonin (Slominski et al., 2002, FASEB J., 16:896-898). They have further tested by nested RT-PCR the expression of genes encoding the receptors of this cutaneous serotoninergic/melatoninergic metabolic pathway. Whole human skin and normal and pathological cultured skin cells predominantly express genes encoding the 5-HT2B and 5HT7 isoforms of the serotonin receptor. The expression of other serotonin receptors isoforms is less prevalent and 5HTR2C rarely detected (Slominski et al., 2003, J. Cell. Phys., 196:144-153).

Members of the ADAR (adenosine deaminases acting on RNA) gene family are involved in one type of RNA editing that convert adenosine residues to inosine. The process of RNA editing is a widespread phenomenon in eukaryotes that leads to posttranscriptional base changes in mRNA. In mammals, a growing number of genes have been identified that undergo a type of RNA editing that is characterized by site-selective adenosine-to-inosine modification.

Among A-to-I editing substrates are the brain-specific transcripts coding for the glutamate receptors AMPA type (such as GluR2 and GluR4) and G-protein-coupled serotonin receptors (such as 5HTR2C). In GluR subunit B (GluR-B), a single editing position (the Q/R-site) controls the Ca2+-permeability of the ion channel, whereas another position (the R/G-site) regulates the desensitization kinetics of the receptor. This property of AMPA receptors is critical for normal brain function. Because of the importance of accurate RNA editing for normal brain function, the deregulation of editing activity may influence the progression of pathophysiological processes, such as neurodegenerative diseases or tumorigenesis (epilepsie cognitif, tumors, sleep waking, mood disorders eating (Maas S et al. (1996) J. Biol. Chem 271, 12221-26; Sergeeva OA et al. (2007) Cell Mol Neurobiol. 27:669-680.

Another ADAR A-to-I editing substrate which has been identified at the level of T cells as an isoenzyme of phosphodiesterases, is the phospodiesterase 8A1. 6 to 7 sites of editing have been identified and could be modulated in pathological state (lupus erythematosus) and after drug action (interferon alpha).

It is important to note that this isoenzyme is present in brain (Orlowski RJ et al., 8A1 gene transcripts of systemic lupus erythematosus T lymphocytes. Immunology 2008 in press; Wang P et al., phosphodiesterase 8A (PDEA8) splice variants: cloning, gene organization and tissue distribution. (2001) Gene 280 183-194).

Among these ADAR brain-specific substrates, the A-to-I editing of 5HTR2C mRNA leads to replacement of three amino acid residues located within the intracellular loop II domain, resulting in dramatic alterations in G-protein coupling functions of the receptor (Yang W et al., Brain Res Mol Brain Res. 2004, 124(1):70-78). Four A-to-I RNA editing enzymes, termed ADAR1, ADAR2, ADAR3 and ADAT1, have been cloned from mammals. ADAR1 isoforms and ADAR2 are widely expressed in a variety of cells and tissues with the highest expression in the brain and spleen and are the essential ADARs involved in 5HTR2C mRNA editing (ADAR 3 was identified solely in the brain and its deaminase activity has not yet been established and ADAT1 targets tRNA). 5HTR2C mRNA is edited at five closely spaced adenosine residues (termed A, B, C, D and E editing sites) allowing the generation of 32 different mRNA variants and 24 different protein isoforms of the receptor ranging from the unedited Ile156-Asn158-Ile160 (INI) isoform to the fully edited Val156-Gly158-Val160 (VGV) isoform. It is known that ADAR1 alone is involved in the A and B editing sites, both ADAR1 and ADAR2 in the E and C editing sites, and ADAR2 alone in the D editing site (Dracheva et al., Molecular Psychiatry, 2007, 1-10).

It is known that interferon-alpha (IFN-alpha) often causes severe depression in patients treated for chronic viral hepatitis and certain malignancies. The effects of IFN-alpha on RNA editing in human glioblastoma cell lines has been observed (Yang et al., Beyond the Identification of Transcribed Sequences: Functional, Evolutionary and Expression Analysis, 12th International Workshop, October 25-28, 2002, Washington, DC, Intracellular Trafficking of A Few Inflammation-Inducible ADAR1 Isoform). It has been also observed, in vivo in the Balb/c Mouse, that the administration of interferon alpha, known to be a powerful activator of the expression of ADAR1 150 *in vitro* in human glioblastoma cells lines (Yang W. et al., 2004), induces also subsequent changes in the editing profile of the 5HTR2C in the dorsal prefrontal cortex.

In order to allow rapid and validated predictive parameters of general modifications of the editing process, it remains desirable:

- to provide a method allowing to extrapolate alterations of the editing rate or profile of these ADAR substrates, particularly the glutamate receptors AMPA type or the 5HTR2C mRNA, in human brain tissue from a biological sample which can be obtained easily from the patient to be tested, and wherein, it will be preferred that the editing rate or profile of these ADAR substrates determined in this biological sample could be correlated with this obtained from brain biological sample; and/or

- to identify a marker present in a biological sample which can be obtained easily from the patient to be tested, wherein the qualitative and/or quantitative analysis of said marker in said sample can be correlated to an alteration of the editing rate or profile of these ADAR substrates in brain tissue, such biological sample and associated marker could be used as a reporter of the receptor editing observed in CNS (central nervous system).

This is precisely the subject of the present invention.

The present invention relates to the use of or to a method implementing a biological sample consisting of peripherical tissues containing cells for evaluating the pathological alteration of the ADAR dependent A to I mRNA editing of the 5HTR2C in the brain , wherein said peripherical tissue containing cells is a mammal skin sample.

The present invention also relates to the use of or to a method implementing a single biological sample or two different biological samples selected from the group of biological sample consisting of peripherical tissues containing cells for evaluating the pathological alteration of a the ADAR dependent A to I mRNA mRNA editing of the 5HTR2C in the brain

The present invention also relates to the use or to a method implementing as a single or an associated reporter sample for evaluating the pathological alteration of said mRNA editing in the brain, of:

- a first peripherical tissue containing cells, such as a skin sample from a mammal; or/and

- a second peripherical tissue containing cells, such as a blood sample from a mammal.

In a preferred embodiment, said edited mRNA whose editing is an ADAR dependent A to I mRNA editing, is a mRNA selected from the group consisting of the mRNA coding for a glutamate receptor AMPA type, for a G-protein-coupled serotonin receptor and for the PDEA8.

In a preferred embodiment, the evaluation of the pathological alteration of the mRNA editing in the brain is determining by:

- the editing rate(s) or profile of the edited forms of said mRNA in said peripherical biological sample; and/or
- the nature or/and the quantity of the ADARs expressed in said peripherical biological sample.

In a more preferred embodiment, said mRNA having an ADAR dependent A to I mRNA editing is the 5HTR2C mRNA.

The inventors have found that the measure of changes in ADARs expression at the periphery (such in skin and optionally in blood) could predict important alteration of editing in the brain.

The inventors have demonstrated for example that the determination of the 5HTR2C mRNA editing and/or the determination of the ADARs activities expressed in peripherical tissue containing cells expressing the 5HTR2C and/or ADARs, such as skin and optionally in a blood tissue sample, can be used as reporter markers of the alteration of the mechanism of the 5HTR2C mRNA editing in the brain tissue and thus, for evaluating the pathological alteration of the multistep-metabolic pathway of the serotinergic system expressed in the CNS.

The inventors have for example demonstrated that the essential ADARs responsible of the editing of the mammal 5HTR2C, which are the two ADAR1 isoforms (named hereinafter "ADAR1-150" and "ADAR1-110" for ADAR1-150 kD protein and ADAR1-110 kD protein) and ADAR2, are all expressed in sufficient quantity in said peripherical tissue containing cells, particularly in skin sample and in blood sample white cells, in order to be qualitatively and/or quantitatively analysed and to use this peripherical tissue containing cells and the ADARs as a reporter sample and marker for evaluating the pathological alteration of the multistep-metabolic pathway of the serotinergic system expressed in the CNS.

They have also demonstrated that contrary to what it has been indicated in the prior art for skin sample (Slominski et al., 2003, J. Cell Phy., 196, 144-153), certain peripherical tissue containing cells, such as skin sample cells, express sufficient 5HTR2C mRNA to be detected and analysed to evaluate the editing rate or profile of the 5HTR2C mRNA, and optionally, the nature and/or the quantity of the ADARs contained.

Consequently,

- the determination of an altered or normal expression (in nature and/or in quantity) of the ADARs enzymes, particularly ADAR1 isoforms (particularly the "ADAR1-150" and "ADAR1-110") and ADAR2, in peripherical tissue containing cells expressing these ADARs, such as in skin and optionally blood tissue sample containing cells; and/or
- the determination of the 5HTR2C mRNA editing rate or profile of the edited forms of the 5HTR2C mRNA in peripherical tissue containing cells expressing the 5HTR2C mRNA, such as in skin and optionally in blood tissue sample containing cells,

can be used as reporter markers of the alteration of the mechanism of the 5HTR2C mRNA editing in the brain tissue and thus, for evaluating the pathological alteration of the multistep-metabolic pathway of the serotinergic system expressed in the CNS of a patient.

Finally, the determination of an altered or normal expression of the ADARs enzymes, alone or in association with the determination of the 5HTR2C mRNA editing rate or profile of the edited forms of the 5HTR2C mRNA, in one or more peripherical tissue samples containing cells, such as in skin and optionally in blood tissue sample containing cells, can be used as reporter sample(s) and markers:

- to identify *in vitro* whether a patient presents a pathology or is at risk to develop a pathology related to an alteration of the mechanism of the mRNA editing of the serotonin 2C receptor (5HTR2C);
- to determine *in vitro* whether a pathology exhibited by a patient is related to an alteration of the mechanism of the mRNA editing of the 5HTR2C;
- to select a compound capable of modulating the 5HTR2C mRNA editing in the brain tissue, preferably compound able to restore the normal 5HTR2C mRNA editing in the brain tissue of a patient in need thereof; or
- to determine *in vitro* in a mammal the efficiency of a drug used for the prevention or for the treatment of a pathology related to an alteration of the mechanism of the mRNA editing of the 5HTR2C.

By "peripherical tissue" containing cells, it is intended to designate herein tissue other than brain tissue and which is preferably easy to collect, such as in general biopsy of organ or tissue easy to collect, skin sample, whole blood sample, urine sample, saliva sample, internal cheek tissue sample, vagina or internal cheek exfoliative cytology or smear. Skin and optionally blood sample containing cells is the preferred peripherical tissue sample implementing in the present invention.

By association of these two markers (ADARs and 5HTR2C mRNA editing), it is intended that the ADARs expression can be analysed in the same type of cells as the cells used for the determination of the 5HTR2C mRNA editing, for example in skin cells sample), or that the ADARs expression can be analysed in one type of cells, for example blood cells sample, and the determination of the 5HTR2C mRNA editing is carried out in another type of cells, for example in skin cells sample.

Each marker can be used also alone whether the determination of this marker is sufficient to correlate its expression in a peripherical tissue samples containing cells, such as in skin and optionally in blood tissue sample containing cells, with the editing rate or profile of the 5HTR2C mRNA in brain tissue.

For another example, the determination of the 5HTR2C mRNA editing rate or profile of the edited forms of the 5HTR2C mRNA or the determination of an altered or normal expression of the ADARs in skin tissue sample containing cells expressing the 5HTR2C mRNA and ADARs, can be also alone as a reporter marker of the alteration of the mechanism of the 5HTR2C mRNA editing in the brain whether the correlation obtained is sufficient with the single marker used.

Concerning the method to select compound capable of modulating the 5HTR2C mRNA editing, the cells of the PeriphericalPeripherical tissue expressing the 5HTR2C mRNA and/or ADARs for evaluating and selected such compounds can be cells lines or recombinant cell lines wherein the expression of the 5HTR2C mRNA and/or ADARs have been altered in order, for example, to mimic pathological expression of this 5HTR2C mRNA and/or ADARs. Skin or optionally blood recombinant cells or cell lines can be particularly used for this aspect.

In particular, the present invention comprises the use of one PeriphericalPeripherical tissue expressing the 5HTR2C mRNA and/or ADARs, such as skin sample (skin cells or tissue, or biopsy) from a mammal, preferably a human, a mouse or a rat, as a single or associated reporter sample for evaluating the pathological alteration of the 5HTR2C mRNA editing system expressed in the CNS, such in brain.

More particularly, the present invention comprises the use of one pheripherical tissue expressing the 5HTR2C mRNA and/or ADARs, such as skin sample from a mammal, preferably a human, a mouse or a rat, as a single or associated reporter sample for evaluating the pathological alteration of the 5HTR2C mRNA editing system expressed in the CNS, such in brain, by:

- determining the editing rate(s) or profile of the edited forms of the 5HTR2C mRNA in that peripherical tissue sample, such as skin cells; or, optionally and if used as associated marker,
- determining the nature or/and the quantity of the ADARs expressed in said peripherical tissue sample.

In another particular embodiment, the present invention comprises the use of a second peripherical tissue sample, such as whole blood sample from a mammal, preferably a human, a mouse or a rat blood sample, more preferably white cells, as a single or associated reporter sample for evaluating the pathological alteration of the 5HTR2C mRNA editing system expressed in the CNS, such in brain.

More particularly, the present invention comprises the use of said second peripherical tissue, such as whole blood sample from a mammal, preferably a human, a mouse or a rat peripherical tissue sample, preferably white cells, as a single or associated reporter sample for evaluating the pathological alteration of the 5HTR2C mRNA editing system expressed in the CNS, such in brain, by:

- determining the editing rate(s) or profile of the edited forms of the 5HTR2C mRNA in that second peripherical tissue sample, such as blood cells; or, optionally and if used as associated marker,
- determining the nature or/and the quantity of the ADARs expressed in said second peripherical tissue sample.

In a first aspect, the present invention is directed to the use of a method according to the invention for identifying *in vitro* whether a patient presents a pathology or is at risk to develop a pathology related

to an alteration of the mechanism of the mRNA editing of the 5HTR2C, wherein this method comprising the following steps of:

a) obtaining from the patient to be tested a biological sample containing peripherical tissue containing cells, such as skin cells, and optionally of blood cells;

b) determining the editing rate for at least one of the edited forms or for the unedited form, of said 5HTR2C mRNA and/or the nature or/and the quantity of the ADARs expressed in said sample of peripherical tissue containing cells, such as skin cells and optionally of blood cells;

c) identifying whether said patient presents or is at risk to develop such a pathology by comparing the editing rate obtained in step b) for this edited or unedited form of said 5HTR2C mRNA and/or by comparing the nature or/and the quantity of the ADARs expressed in said sample peripherical tissue containing cells, with characteristic control editing rates of the 5HTR2C mRNA or expressed ADARs profile obtained for normal patients or for patients exhibiting pathologies related to an alteration of the mechanism of this mRNA editing.

In a preferred embodiment, said pathology is selected from the group consisting of mental disorders, schizophrenia, depression, depressed suicide or abnormal feeding behaviour.

In a second aspect, the invention relates to the use of a method according to the invention for determining *in vitro* whether a pathology exhibited by a patient is related to an alteration of the mechanism of the mRNA editing of the 5HTR2C, wherein this method comprising the following steps of:

a) obtaining from the patient exhibiting said pathology a biological sample containing peripherical tissue containing cells, such as skin cells, and optionally of blood cells;

b) determining the editing rate for at least one of the edited forms or for the unedited form, of said 5HTR2C mRNA and/or the nature or/and the quantity of the ADARs expressed in said sample of peripherical tissue containing cells, such as skin cellsand optionally blood cells;

c) identifying whether said patient presents or is at risk to develop such a pathology by comparing the editing rate obtained in step b) for this edited or unedited form of said 5HTR2C mRNA and/or by comparing the nature or/and the quantity of the ADARs expressed in said peripherical tissue sample with characteristic control editing rates of the 5HTR2C mRNA or expressed ADARs profil obtained from normal patients or from patients exhibiting pathologies known to be not related to an alteration of the mechanism of this mRNA editing.

In a general aspect, the present invention is directed to:

- the use of a method according to the invention for identifying *in vitro* whether a patient presents a pathology or is at risk to develop a pathology related to an alteration of the mechanism of the editing of a mRNA whose editing is A to I editing ADAR dependent in a mammal;
- the use of a method according to the invention for identifying *in vitro* an agent that modulates the editing of a mRNA whose editing is A to I editing ADAR dependent in a mammal;
- the use of a method according to the invention for determining *in vitro* in a patient the efficiency of a drug used for the prevention or for the treatment of a pathology related to an alteration of the mechanism of the editing of a mRNA whose editing is A to I editing ADAR dependent in a mammal;
- the use of a method according to the invention for determining if a patient responds or does not respond to a treatment of a pathology resulting or provoking by the alteration of the mechanism of the editing of a mRNA whose editing is A to I editing ADAR dependent in a mammal,

wherein this method comprises the following steps of:

a) obtaining from the patient to be tested a peripherical biological sample containing cells;

b) determining the nature or/and the quantity of the ADARs expressed in said peripherical biological sample;

c) comparing the nature or/and the quantity of the ADARs expressed in said sample with characteristic control of expressed ADARs profil obtained for normal patients or for patients exhibiting pathologies related to an alteration of the mechanism of this mRNA editing.

In a preferred embodiment for these above methods of the present invention, said edited mRNA is a mRNA selected from the group consisting of the mRNA coding for a glutamate receptor AMPA type, for a G-protein-coupled serotonin receptor and for the PDEA8.

In a third aspect, the invention is directed to the use of a method according to the invention for identifying *in vitro* an agent that modulates *in vivo* the editing of the 5HTR2C mRNA in a mammal, comprising the following steps of:

a) administering to said mammal a candidate modulator of the 5HTR2C mRNA editing;

b) obtaining from said mammal a biological sample containing peripherical tissue containing cells, such as skin cells; and

c) determining the effects of said modulator:

- on the editing rate of at least one of the edited or unedited forms of said 5HTR2C mRNA; and/or
- on the nature or/and the quantity of the ADARs expressed in said peripherical tissue sample, such as skin cells and optionally blood cells,

by comparing the editing rate for this edited or unedited form and/or the nature or/and the quantity of the ADARs expressed obtained from the biological sample in step b) with the editing rate and/or the nature or/and the quantity of the ADARs expressed obtained from control peripherical tissue containing cells of said mammal.

In this third aspect, the invention also comprises the use of a method according to the invention for identifying *in vitro* an agent that modulates the editing of the 5HTR2C mRNA in a mammal, comprising the following steps of:

a) obtaining a biological sample containing mammalian peripherical tissue containing cells, such as skin cells line, optionally, these cells can be recombinant cells or cells lines;
b) contacting said biological sample in the presence of a candidate modulator of said 5HTR2C mRNA editing; and
c) determining the effects of said modulator:

-    on the editing rate of at least one of the edited or unedited forms of said 5HTR2C mRNA; and/or
-    on the nature or/and the quantity of the ADARs expressed in said peripherical tissue sample,

by comparing the editing rate for this edited or unedited form and/or the nature or/and the quantity of the ADARs expressed obtained from the biological sample in step b) with the editing rate and/or the nature or/and the quantity of the ADARs expressed obtained from control peripherical tissue containing cells, such as skin cells and optionally bloods cells, of said mammal.

In this third aspect, the invention also comprises the implementation of these above methods to detect alterations of the editing processes of regulation induced by a treatment such as antidepressants, antipsychotics, anti-obesity, anti-viral infection, ... treatments which have been identified to present a significant action on brain editing regulation and trigger identified risks such as suicide, resistance to treatment, induced chronicity.

In a fourth aspect, the invention comprises the use of a method according to the invention for determining *in vitro* in a mammal the efficiency of a drug used for the prevention or for the treatment of a pathology related to an alteration of the mechanism of the mRNA editing of the 5HTR2C, comprising the following steps of:

a) obtaining from said mammal a biological sample containing peripherical tissue containing cells, such as skin cells, and optionally blood cells, and determining the editing rate for at least one of the edited forms or for the unedited form, of said 5HTR2C mRNA and/or the nature or/and the quantity of the ADARs expressed in said peripherical tissue sample, such as in skin cells and optionally blood cells;
b) administering to said mammal the drug intended for the prevention or for the treatment of a pathology;
c) obtaining from said mammal during or/and after the treatment a new sample of said peripherical tissue sample and determining the editing rate for at least one of the edited forms or for the unedited form, of said 5HTR2C mRNA and/or the nature or/and the quantity of the ADARs expressed in said sample chosen in step a); and
d) determining the efficiency of said drug by comparing the editing rate and/or the nature or/and the quantity of the ADARs expressed obtained from the biological sample in step a) with this obtained in step c), a modulation of the editing rate and/or the nature or/and the quantity of the ADARs expressed resulting to an editing rate and/or a nature or/and a quantity of the ADARs expressed close or equal to this observed for normal patients being significant of the efficiency of the treatment.

In this aspect, the present invention relates to the use of a method according to the invention to determining if a patient responds or does not respond to a treatment of a pathology resulting or
provoking by the alteration of the mechanism of the mRNA editing of the 5HTR2C, further comprising a steps of:

e) determining if the patient responds or not responds to the treatment by observing the modification of the editing rate(s) or profile and/or the nature or/and the quantity of the ADARs expressed after a period of treatment (i.e. 15 days, 30 days, 2 months, 6 months, etc.) by comparing with the editing rate(s) or profile and/or the nature or/and the quantity of the ADARs expressed before the beginning of the treatment.

Such a method allows to avoid to extend needlessly the period of treatment with a drug if it can be thus possible to determine rapidly that the patient does not respond to that drug, or to continue the treatment if the patient responds to that drug.

Editing is the mechanism by which information contained in the gene is modified after transcription. The general term "mRNA editing" includes the modification of the sequence of these mRNAs which results in a change, in terms of nature or number, in the amino acids incorporated into the protein during translation, it no longer being possible for the sequence of the protein to be deduced from that of the gene which directs its synthesis. The pre-messenger RNA of 5HTR2C can undergo a specific enzymatic modification of certain adenosines (A), in the portion of what will become the definitive mRNA which directs the incorporation of the amino acids located in the second intracellular loop of 5HTR2C. In fact, the distal part of the fifth exon and the proximal part of the fifth intron of the primary transcript are capable of forming a stem-loop structure potentially recognized by two enzymes, ADAR1 and ADAR2 (double-stranded RNA-dependent adenosine deaminase), which make it possible to edit the premessenger RNA before it is spliced. This editing is produced by deamination of As, which are then converted to inosine (I). Once the splicing has been completed, the part of the mRNA which contained the As which underwent the editing now contains Is. When the 5HTR2C mRNA is translated, it is thought that the Is are read as Gs. In fact, during *in vitro* synthesis of the cDNA from the 5HTR2C mRNA that underwent the deamination of As to Is, the reverse transcriptase incorporates dCs opposite the Is, instead of dTs which should normally have been incorporated opposite the As. Consequently, during the synthesis of the second strand which results in the formation of the double-stranded cDNA, a dG is introduced opposite each dC incorporated into the first strand. Sequencing of the double-stranded cDNA thus obtained makes it possible to observe the replacement of the dAs with dGs, due to the initial deamination of the As to Is in the mRNA which underwent the editing. Consequently, the editing of the mRNA results in a modification of the meaning of the codons in which the As are replaced with Is, which are therefore thought to be read as Gs (more specifically, with regard to the editing of human 5HTR2C, see Fitzgerald et al., Neuropsychopharmacology, 1999, 21(2S), 82S-90S).

So, the determination of an alteration of the mechanism of the mRNA editing of the 5HTR2C by the control of the editing rate in skin sample is also significant of an alteration of the multistep-metabolic pathway of the serotoninergic system expressed in the skin and which could be used as a reporter of the serotoninergic system expressed in brain.

Thus, in a sixth aspect, the present invention also comprised a method to control the alteration of the acting mechanism of proteins which are involved in the mRNA editing of the 5HTR2C, such as the ADAR1 and/or ADAR2 enzymes, by determining the edition rate of this 5HTR2C mRNA from a peripherical tissue containing cells, such as skin sampleor optionally blood sample, by the method for determining the editing rate(s) of the edited or unedited forms of said 5HTR2C mRNA as implemented or described in the above methods according to the present invention.

It is important to note that the present invention is directed to the use of peripheral markers of the editing process to diagnose and follow the general alterations of its regulation with a predictable implication in pathologies which alter brain and/or peripheral functions. The principal goal is, as a non exclusive example, to reach a new capacity to predict and orientate the therapy in patients for whom an alteration of the editing regulation has been suggested to participate to their pathology (e.g. as in depression and suicide) either after convergent post-mortem observations or indirect clinical evidence (e.g. as depressive state induced by interferon treatment) (See particularly Example 1 for the strategy implemented for that goal).

The term "edited RNA" is intended to denote, in the present description, any RNA sequence in which at least one adenosine has been deaminated to inosine by an adenosine deaminase.

By "editing rate", it is intended to designate the percentage of each of the edited and unedited forms of the mRNA which may comprise at least one editing site, relative to the total amount of the edited or unedited mRNA forms present in said same sample.

| | Editing sites | | A B | EC | D | |
|---|---|---|---|---|---|---|
| 1 | 5HTR2C-0 | I-N-I | ATACGTAATCCTATT | | | SEQ ID No. 1 |
| 2 | 5HTR2C-A | V-N-I | ITACGTAATCCTATT | | | SEQ ID No. 2 |
| 3 | 5HTR2C-B | M-N-I | ATICGTAATCCTATT | | | SEQ ID No. 3 |
| 4 | 5HTR2C-C | I-S-I | ATACGTAITCCTATT | | | SEQ ID No. 4 |
| 5 | 5HTR2C-D | I-N-V | ATACGTAATCCTITT | | | SEQ ID No. 5 |
| 6 | 5HTR2C-E | I-D-I | ATACGTIATCCTATT | | | SEQ ID No. 6 |
| 7 | 5HTR2C-AB | V-N-I | ITICGTAATCCTATT | | | SEQ ID No. 7 |
| 8 | 5HTR2C-AC | V-S-I | ITACGTAITCCTATT | | | SEQ ID No. 8 |
| 9 | 5HTR2C-AD | V-N-V | ITACGTAATCCTITT | | | SEQ ID No. 9 |

(continued)

| | Editing sites | A B | EC | D | |
|---|---|---|---|---|---|
| 10 | 5HTR2C-AE V-D-I | ITACGTIATCCTATT | | | SEQ ID No. 10 |
| 11 | 5HTR2C-BC M-S-I | ATICGTAITCCTATT | | | SEQ ID No. 11 |
| 12 | 5HTR2C-BD M-N-V | ATICGTAATCCTITT | | | SEQ ID No. 12 |
| 13 | 5HTR2C-BE M-D-I | ATICGTIATCCTATT | | | SEQ ID No. 13 |
| 14 | 5HTR2C-CD I-S-V | ATACGTAITCCTITT | | | SEQ ID No. 14 |
| 15 | 5HTR2C-CE I-G-I | ATACGTIITCCTATT | | | SEQ ID No. 15 |
| 16 | 5HTR2C-DE I-D-V | ATACGTIATCCTITT | | | SEQ ID No. 16 |
| 17 | 5HTR2C-ABC V-S-I | ITICGTAITCCTATT | | | SEQ ID No. 17 |
| 18 | 5HTR2C-ABD V-N-V | ITICGTAATCCTITT | | | SEQ ID No. 18 |
| 19 | 5HTR2C-ABE V-D-I | ITICGTIATCCTATT | | | SEQ ID No. 19 |
| 20 | 5HTR2C-ACD V-S-V | ITACGTAITCCTITT | | | SEQ ID No. 20 |
| 21 | 5HTR2C-ACE V-G-I | ITACGTIITCCTATT | | | SEQ ID No. 21 |
| 22 | 5HTR2C-ADE V-D-V | ITACGTIATCCTITT | | | SEQ ID No. 22 |
| 23 | 5HTR2C-BCD M-S-V | ATICGTAITCCTITT | | | SEQ ID No. 23 |
| 24 | 5HTR2C-BCE M-G-I | ATICGTIITCCTATT | | | SEQ ID No. 24 |
| 25 | 5HTR2C-BDE M-D-V | ATICGTIATCCTITT | | | SEQ ID No. 25 |
| 26 | 5HTR2C-CDE I-G-V | ATACGTIITCCTITT | | | SEQ ID No. 26 |
| 27 | 5HTR2C-ABCD V-S-V | ITICGTAITCCTITT | | | SEQ ID No. 27 |
| 28 | 5HTR2C-ABCE V-G-I | ITICGTIITCCTATT | | | SEQ ID No. 28 |
| 29 | 5HTR2C-ABDE V-D-V | ITICGTIATCCTITT | | | SEQ ID No. 29 |
| 30 | 5HTR2C-ACDE V-G-V | ITACGTIITCCTITT | | | SEQ ID No. 30 |
| 31 | 5HTR2C-BCDE M-G-V | ATICGTIITCCTITT | | | SEQ ID No. 31 |
| 32 | 5HTR2C-ABCDE V-G-V | ITICGTIITCCTITT | | | SEQ ID No. 32 |

\* The editing site " E " is also named " C' "

In a preferred embodiment of the methods according to the invention, the patient or the mammal is human, a mouse or a rat, preferably a human.

In a preferred embodiment of the methods according to the invention, the skin cells are selected from the group consisted of keratinocytes, melanocytes, fibroblasts, Langerhans cells and Merkels cells, and the skin tissue is selected from the group consisted of epidermis and dermis.

The keratinocytes can be from human immortalized cells, such as HaCaT cells line, or the melanocytes are from human immortalized cells or melanoma.

In a preferred embodiment of the methods according to the invention, the keratinocytes are from neonatal for skin, dermis or hair follicles, melanocytes are from epidermis or from hair follicles, and fibroblasts are from dermis or papillary hair follicles.

In a more preferred embodiment of the methods according to the invention, the skin cells, cultured skin-derived cells or skin tissue are from eyelid or auricular skin.

In a preferred embodiment of the methods according to the invention, the edition sites of said 5HTR2C mRNA are selected from the nucleotides localized in position 1, 3, 7, 8 and 13 of the human 5HTR2C mRNA fragment having the sequence 5'-AUA CGU AAU CCU AUU-3' (SEQ ID No. 33).

In a preferred embodiment of the methods according to the invention, the editing rate is determined for at least 1, preferably 2, 3, 4, 5, 6, 7, 8, 9, 10, 11, 12, 13, 14, 15, 16, 17, 18, 19, 20, 2, 22, 23, 24, 25, 26, 27, 28, 29, 30 or 32 of the edited and unedited forms of the human 5HTR2C mRNA.

In a more preferred embodiment, the editing rate is determined for all the edited and unedited forms of said 5HTR2C mRNA (32 forms).

In a preferred embodiment of the methods according to the invention, the editing rate for each edited and unedited form of said 5HTR2C mRNA is determined by a method which comprises the following steps:

A) extraction of the total RNAs of said skin cells sample, such as skin cells line, cultured skin-derived cells or skin tissue, or optionally of said blood cells, such as white cells, followed, where appropriate, by purification of the mRNAs;
B) reverse transcription of the RNAs extracted in step A); and
C) PCR amplification of the cDNAs obtained in step B) using at least a pair of primers specific for the 5HTR2C mRNA fragment containing the edition sites which may be edited, this pair of primers being chosen so as to be able to amplify all the editing forms and the unedited form potentially present in the RNA extract.

In a more preferred embodiment of the methods according to the invention the editing rate for each edited and unedited form of said 5HTR2C mRNA is determined by a method which comprises the following steps:

A) extraction of the total RNAs of said skin cells sample, such as skin cells line, cultured skin-derived cells or skin tissue, or optionally of said blood cells sample, such as white cells, followed, where appropriate, by purification of the mRNAs;
B) reverse transcription of the RNAs extracted in step A); and
C) PCR amplification of the cDNAs obtained in step B) using at least a pair of primers specific for the 5HTR2C mRNA fragment containing the edition sites which may be edited, this pair of primers being chosen so as to be able to amplify all the editing forms and the unedited form potentially present in the RNA extract, and wherein the step B) of reverse transcription is carried out by using an oligonucleotidic primer specific of the 5HTR2C gene.

In a more preferred embodiment of the step B), the oligonucleotidic primer specific of the 5HTR2C gene has the sequence 5'-TTCGTCCCTCAGTCCAATCAC-3' (SEQ ID No. 34).

In a preferred embodiment of the methods according to the invention, in step C), the PCR amplification step is a nested type PCR comprising two rounds of PCR, and wherein the first round of PCR is carried out by a set of primers which results to a PCR nucleic acid product having a length comprised between 200 bp and 300 bp, preferably between 225 bp and 275 bp, more preferably between 240 bp and 260 bp, 250 bp is the most preferred.

In a more preferred embodiment of the methods according to the invention in step C), the PCR amplification step is a nested type PCR comprising two rounds of PCR, and wherein the second round of PCR is carried out by a set of primers which results to a final PCR nucleic acid product having a length comprised between 90 bp and 160 bp, preferably between 100 bp and 140 bp, more preferably between 110 bp and 140 bp. A final PCR product having a sequence length between 110 bp and 138 bp is the most preferred.

In an also more preferred embodiment of the methods according to the invention, in step C), the PCR amplification step is a nested type PCR comprising two rounds of PCR, and wherein the first round of PCR is carried out by the following set of primers:

for human:

PCR9 Forward: 5'-TGTCCCTAGCCATTGCTGATATGC-3', SEQ ID No. 35;

PCR10 Reverse: 5'-GCAATCTTCATGATGGCCTTAGTC-3', SEQ ID No. 36; and

for mouse or rat:

PCR9 Forward: 5'-TGTCCCTAGCCATTGCTGATATGC-3', SEQ ID No. 35;
PCR10 Reverse: 5'-GCAATCTTCATGATGGCCTTAGTC-3', SEQ ID No. 36,

wherein the second round of PCR is carried out by the following set of primers:

for human:

PCR18 Forward: 5'-ATGTGCTATTTTCAACAGCGTCCATC-3', SEQ ID No. 37;
PCR2 Reverse: 5'-GCAATCTTCATGATGGCCTTA-3', SEQ ID No. 38; and

for mouse or rat:

PCR1 Forward: 5'-TTTGTGCCCCGTCTGGAT-3', SEQ ID No. 39;
PCR4 Reverse: 5'-GCCTTAGTCCGCGAATTG-3', SEQ ID No. 40.

The two followed set of primers used for amplifying by nested PCR all the isoforms of the human edited and unedited human 5HTR2C mRNA are included in the present invention, preferably, the set of primers used for the second of PCR:

first round:

PCR9 Forward: 5'-TGTCCCTAGCCATTGCTGATATGC-3' (SEQ ID No. 35);
PCR10 Reverse: 5'-GCAATCTTCATGATGGCCTTAGTC-3' (SEQ ID No. 36); and

second round:

PCR18 Forward: 5'-ATGTGCTATTTTCAACAGCGTCCATC-3' (SEQ ID No. 37);
PCR2 Reverse: 5'-GCAATCTTCATGATGGCCTTA-3' (SEQ ID No. 38).

The primers used in the PCR amplification step if there is one round of PCR, or used in the second round if it is a nested type PCR having two round of PCR, are preferably labelled, more preferably labelled with fluorophores, such as C6-FAM (MWG) or VIC (Applied Biosystem).

In an also more preferred embodiment of the methods according to the invention, the editing rate for each edited and unedited form of said 5HTR2C mRNA is determined by an SSCP method capable of providing the editing profile for each of the edited and unedited separate forms of said mRNA, said SSCP method being characterized in that it comprises after the steps A), B) and C) the following steps:

D) where appropriate, purification of the PCR products obtained in step C);
E) where appropriate, quantification of the PCR products obtained in step D);
F) dissociation of the double-stranded DNAs to single-stranded DNAs, in particular by heating followed by abrupt cooling;
G) separation of the single-stranded DNAs by capillary electrophoresis; and
H) obtaining of the editing profile by reading of the fluorescence and, where appropriate, acquisition of the profile data by means of the exploitation system associated with the fluorescence reader.

The obtaining of the electrophoretic migration profile of the various single-stranded DNAs corresponding to the various edited form of the 5HTR2C cDNA fragment containing the five edition sites is referred to here as the "editing profile".

In a preferred embodiment, the control or standard editing rates or editing profiles of the 5HTR2C mRNA used in step c) of claims 1 to 4 for determining the risk of pathology, the associated pathology to the alteration of the 5HTR2C mRNA editing or the effect of the tested agent, are characteristic editing rates or profiles obtained for each of the edited and unedited separate forms of said mRNA with the same method and under the same given conditions used for the tested biological sample.

In a general way, the quality and/or quantity of each edited and unedited separate form present in the biological sample to be tested can be evaluated by comparison with the edition rates or profiles of known qualitative and/or quantitative mixtures of each of these edited and unedited forms, obtained with the same method, such as the SCCP method described above, and under the same conditions used for the tested biological sample.

In another aspect, the present invention is directed to an isolated nucleic acid wherein this nucleic acid:

- comprises or has the sequence ATGTGCTATTTTCAACAGCGTCCATC (SEQ ID No. 37) and, preferably, has at most 100 nucleotides, more preferably 90, 80, 75, 70, 65, 60, 55, 50, 45, 40, 35, 30, 29, 28, 27 and 26 nucleotides; or
- comprises the fragment nt5-nt14 of SEQ ID N° 37, preferably the fragment nt4-nt14, nt3-nt14, nt2-nt14, nt1-nt14, nt5-nt15, nt5-nt16, nt5-nt17, nt5-nt16, nt5-nt17, nt5-nt18, nt5-nt19, nt5-nt20, nt5-nt21, nt5-nt22, nt5-nt23, nt5-nt24, nt5-nt25 of SEQ ID No. 37.

In a more embodiment, the isolated nucleic acid according to the invention is labelled, preferably with a fluorophore.

In this aspect, the present invention comprises the use of said nucleic acid according to the invention as a primer or a probe, preferably as a primer in a PCR amplification method, more preferably in a nested PCR as a second primer.

The present invention relates to a kit for the determination of a mammal 5HTR2C mRNA editing rate or profile, preferably in human, in rat or in mouse, more preferably in human, wherein said kit contains a nucleic acid according to the invention.

In a preferred embodiment of the methods of the present the cells which are selected for determining in association the nature and/or the quantity of the expressed ADARs are blood white cells or leucocytes or originated from the buffy coat of a whole blood sample obtained after centrifugation.

In a preferred embodiment, in step b), the ADAR expression products are ADAR1, isoforms 150 and/or 110, and the ADAR2 gene expression products, preferably the expression products of the mouse, rat or human gene encoding the ADAR1, isoforms 150-kD and/or 110-kD protein, and the ADAR2 protein. Human is the most preferred.

The ADAR1 mRNA nucleic sequence encoding the protein isoforms 150 kD and 110 kD, and the ADAR2 mRNA nucleic sequence encoding the ADAR2 protein, and its amino acid sequence are well known from the skilled person for the human, mouse or rat.

For example, the following sequences depicted in Genbank under the accession number can be particularly cited:

for ADAR1:

- for Human: NM_001111.3; NM_001025107.1,
- for Mouse: NM_019655.2; NM_001038587.2,

for ADAR2:

- for Human: NM_001112.2; NM_015833.2; NM_015834.2 and NM_001033049.1,
- for Mouse :NM_130895.2; NM_001024837; 1NM_001024838.1; NM_001024840.1 and NM_001024839.1.

In a more preferred embodiment, in step b), the ADAR expression products are the ADAR mRNAs.

Thus, the present invention is directed to a method according the invention, wherein in step b), the determination of the ADAR mRNA is carried out by a method which comprises the following steps:

A) extraction of the total RNAs of said skin sample cells and optionally blood sample cells, followed, where appropriate, by purification of the mRNAs;
B) reverse transcription of the RNAs extracted in step A) via an oligo dT primer; and
C) PCR amplification of the cDNAs obtained in step B) using at least a pair of primers specific for each of the ADAR mRNA to be quantified and/or qualitatively analysed.

In a preferred embodiment, the pair of primers specific for the ADAR mRNA PCR amplification are selected from the group consisting of:

- for human ADAR1-150 isoform mRNA amplification:

EX1A 34p Forward: 5'-GCCTCGCGGGCGCAATGAATCC-3' (SEQ ID No. 41),
EX2 578m Reverse: 5'-CTTGCCCTTCTTTGCCAGGGAG-3' (SEQ ID No. 42);

- for human ADAR1-110 isoform mRNA amplification:

  EX1B 534p Forward: 5'-CGAGCCATCATGGAGATGCCCTCC-3' (SEQ ID No. 43),
  EX2 804m Reverse: 5'-CATAGCTGCATCCTGCTTGGCCAC-3' (SEQ ID No. 44);

- for human ADAR2 mRNA amplification:

  ADAR2 1274p Forward: 5'-GCTGCGCAGTCTGCCCTGGCCGC-3' (SEQ ID No. 45),
  ADAR2 1486m Reverse: 5'-GTCATGACGACTCCAGCCAGCAC-3' (SEQ ID No. 46);

- for mouse ADAR1-150 isoform mRNA amplification:

  EX1A 19p Forward: 5'-GTCTCAAGGGTTCAGGGGACCC-3' (SEQ ID No. 47),
  EX2 646m Reverse: 5'-CTCCTCTAGGGAATTCCTGGATAC-3' (SEQ ID No. 48);

- for mouse ADAR1-110 isoform mRNA amplification:

  EX1B 72p Forward: 5'-TCACGAGTGGGCAGCGTCCGAGG-3' (SEQ ID No. 49),
  EX2 646m Reverse: 5'-CTCCTCTAGGGAATTCCTGGATAC-3' (SEQ ID No. 48); and

- for mouseADAR2 mRNA amplification:

  EX7 1281p Forward: 5'-GCTGCACAGTCTGCCTTGGCTAC-3' (SEQ ID No. 50),
  EX9 1622m Reverse: 5'-GCATAAAGAAACCTGAGCAGGGAC-3' (SEQ ID No. 51);

In a second aspect of the method according to the present invention, in step b), the ADAR expression products are the ADAR proteins.

Thus, in a preferred embodiment of this method, the determination of the ADAR proteins is carried out by a method which comprises the following steps:

A) optionally, the extraction of the total proteins contained in said skin sample cells and optionally blood sample cells, followed, where appropriate, by a step of proteins purification; and
B) the determination of the presence, nature and/or the concentration of each ADAR protein contained in said skin sample cells and optionally blood sample cells by the implementation of antibodies capable of recognizing specifically said ADAR proteins, preferably labelled antibodies.

Among these antibodies which can be used for this detection or/and quantification, the followed antibodies can be cited but are not limited to:

sc-33179 anti-ADAR1 (H-176) polyclonal antibody (Santa-Cruz); or
sc-33180 anti-ADAR2 (H-90) polyclonal antibody (Santa-Cruz).

Western blotting or Elisa method can be used for analysing or quantifying specific protein expression in biological sample. Such methods are well known from the skilled man.

The blots can be detected using antibodies specifically directed against different specific regions or epitope of mouse, human or rat ADAR1-150 or ADAR1-110, and ADAR2 protein. These antibodies can be polyclonal or monoclonal antibodies and are labelled if necessary. Such antibodies can be developed in laboratory using recombinant ADAR protein or fragment thereof as immunogen.

The following examples and also the figures and the legends hereinafter have been chosen to provide those skilled in the art with a complete description in order to be able to implement and use the present invention. These examples are not intended to limit the scope of what the inventor considers to be its invention, nor are they intended to show that only the experiments hereinafter were carried out.

**Legends of the figures**

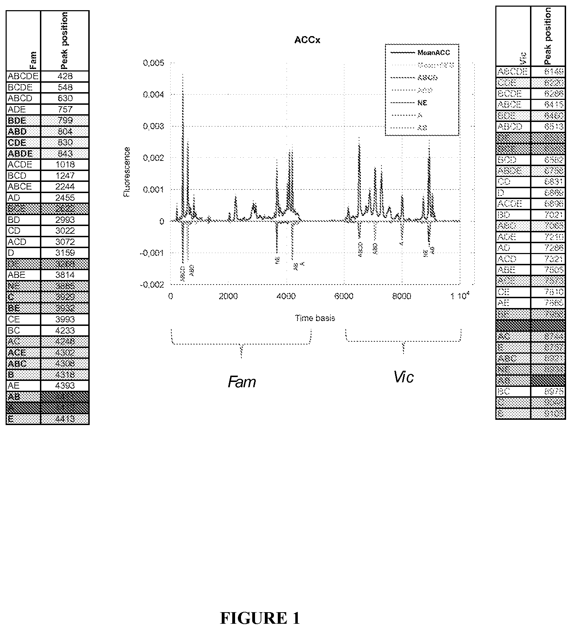

**Figure 1:** Mean (Black line) of electrophoretic SSCP signals calculated from each individual anterior cingulate cortex signals of a controls group of human subjects (experimental data). The curve corresponding to the mean + SEM of these signals is also presented (green line). The abcissae represents the time basis of 10000 points (6.2 points/second) and the ordinates are given in fluorescence arbitrary units after normalization of the total signal. The presented signal corresponds to the FAM (left part) and VIC (right part) labelled strands. Some typical examples of electrophoresis signals obtained with individual standards are presented as negative in their corresponding FAM and VIC labelling. On the left and right parts of the figure the tables present, applicated to an unique base time, the positions of the different principal electrophoresis peaks (maximum) of each standard single strand of a particular edited isoform labelled with FAM (left) or VIC (right) probes. Two maximum of identified peaks could be separated when their interval was $\geq$ 15 points of the base time defined above. Note that some non resolved peaks from electrophoretic pattern of one labelled strand are resolved from the other (these cases are identified by the same color).

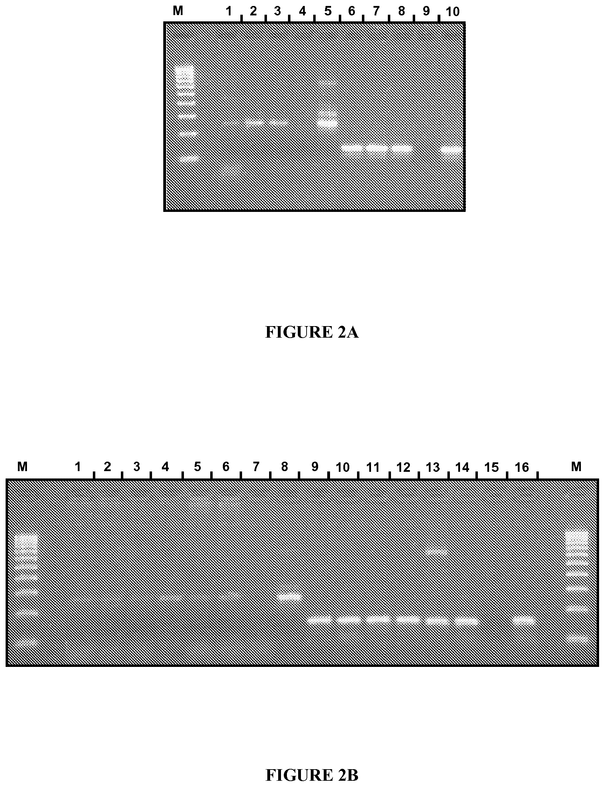

**Figures 2A and 2B:** Identification of 5-HT2cR transcripts in Human and mouse skin. (A) cDNAs prepared from Human eyelid polyA+ RNAs (lanes 1-3) were amplified by a first-round PCR using the two specific primers PCR9 and PCR10. A nested-PCR was then conducted on these first products with primers PCR18 and PCR2 (lanes 6-8). The resulting products were resolved on a 2% agarose gel. The expected sizes of the amplification products are 250-bp and 127-bp long for first and second PCR, respectively. Negative (lanes 4 and 9) and positive controls (lanes 5 and 10) are shown for each primer set. A 100-bp DNA ladder/marker is indicated by M. (B) cDNAs prepared from Balb/c mouse skin polyA+ RNAs were amplified by a first-round PCR using the two gene specific primers PCR9 and PCR10 (lanes 1-6). A second PCR was then conducted on these first amplifications with primers PCR1 and PCR4 (lanes 9-14). Negative (lanes 7 and 15) and positive controls (lanes 8 and 16) are shown for each primer set. The resulting products were resolved on a 2% agarose gel. The expected sizes of the amplification products are 250-bp and 138-bp long for first and second PCR, respectively. M is for the 100-bp DNA ladder/marker.

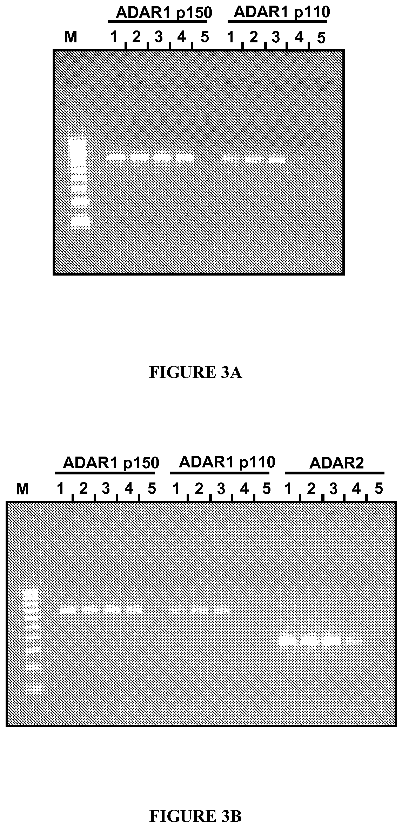

**Figures 3A and 3B:** The mRNA of the ADARs isoenzymes are clearly identified in Balb/c Mouse Blood and Skin. (A) cDNA prepared from whole-blood RNA (lane 4) and skin RNA (lanes 1-3, corresponding to 3 different times of reverse transcription, i.e 30 min, 2 h, and 4 h respectively) were amplified by PCR with primers specific to the constitutive (p110) and inducible forms of ADAR1 (p150). The resulting amplification products resolved on a 2% agarose gel are 674-bp (p150 isoform) and 683-bp (p110 isoform) long, respectively. Negative controls (lane 5) and the 100-bp DNA ladder/marker (M) are shown. The boosted contrast allows detection of a faint band corresponding to the constituve form of ADAR1 transcribed in whole-blood cells (lane 4, p110). (B) Same as in (A), but PCR amplifications corresponding to ADAR2 transcripts in skin and whole-blood are presented. The PCR products are 366-bp long. Again, a very faint band corresponding to the constituve form of ADAR1 in whole-blood is observed (lane 4, p110).

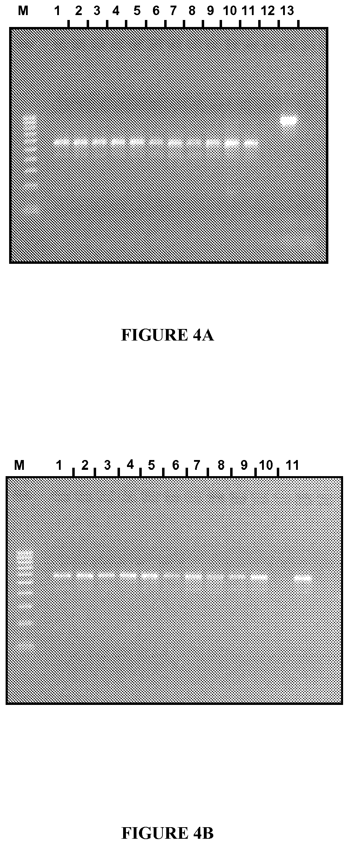

**Figures 4A and 4B:** Identification of inducible ADAR 1 transcripts in Human leukocytes. (A) cDNAs prepared from Human peripheral blood leukocytes total RNA (lane 10) and from Human blood fractions normalized libraries (lanes 1-9) were amplified by PCR with primers specific to the inducible form of ADAR1 (p150). The resulting products were resolved on a 2% agarose gel. The expected size of the amplification product is 544-bp long. Lanes 1 and 6; 2 and 7; 3 and 8 correspond to resting and activated mononuclear cells, resting and activated CD4+, and resting and activated CD8+ cells, respectively. Lane 4 and 5: resting CD14+ and CD19+ cells. Lane 9: activated CD 19+ cells. Lane 11: cDNA from Human placenta used as a control. The 100-bp DNA ladder/marker is indicated by M. PCR positive (G3PDH primers with Human placental cDNA, lane 13) and PCR negative controls (lane 12) are shown. (B) Duplicate of (A). Lanes 1-9 are equivalent to lanes 1-9 of (A). Lane 10: Human placenta cDNA.

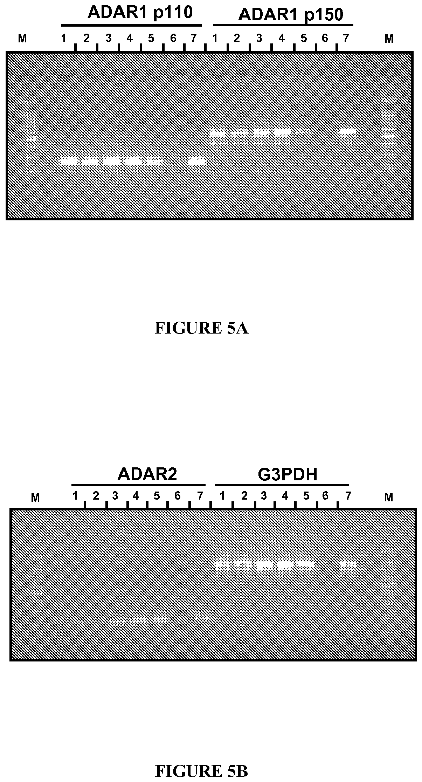

**Figures 5A and 5B:** Identification of ADAR1 p110, ADAR1 p150, and ADAR2 transcripts in Human Dorsal Prefrontal Cortex, skin and CD4+ and CD8+ blood cells. (A) cDNAs from Human CD4+ and CD8+ blood fractions normalized libraries (lanes 1 and 2); prepared from DPFC total RNA (lanes 3 and 4) and from Human eyelid polyA+ RNA (lane 5) were amplified by PCR using the two specific set of primers EX1B 534p/EX2 804m and EX1A 34p/EX2 578m for ADAR1 p110 and ADAR1 p150 respectively. The resulting products were resolved on a 1.75 % agarose gel. The expected sizes of the amplification products are 270-bp (ADAR1 p110) and 566-bp (ADAR1 p150). Negative (lane 6) and positive controls (lane 7, Human placental cDNA) are shown for each primer set. A 100-bp DNA ladder/marker is indicated by M. (B) The same cDNAs as in A) were amplified by PCR using the two gene specific set of primers ADAR2 1274p/ADAR2 1486m and G3PDH-F/G3PDH-R for ADAR2 and G3PDH (positive control) respectively. The resulting products were resolved on a 1.75 % agarose gel Negative. The expected size for ADAR2 is 212-bp long.

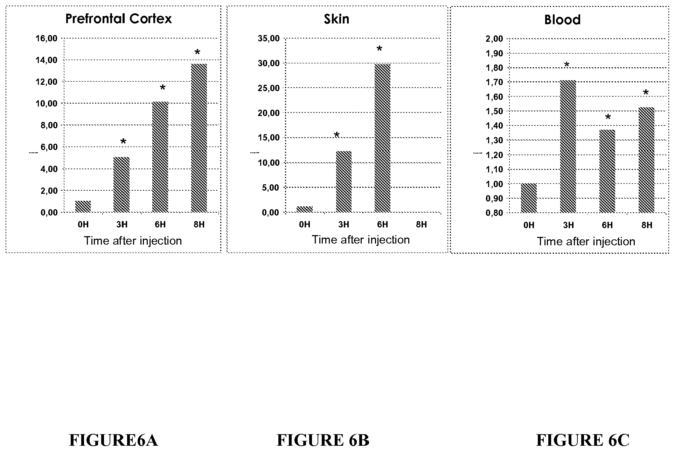

**Figures 6A-6C:** Evolution of the concentration of ADAR1 a specific mRNA measured by QPCR in prefrontal cortex (figure 6A), skin (figure 6B) and blood (figure 6C) sample after interferon alpha2a mouse interferon single IP injection at t zero (20000 IU). The effect is expressed as the fold increase from control value normalized at 1. * p<0.05.

**EXAMPLE 1:** Strategy implemented

The validation of such a strategy has implicated:

1) The identification of significant alteration of the editing process in human brain in a given pathology and the validation of its pathogenic implication in the pathology by post-mortem observations made in convergent pathological models obtained in mouse and/or rat.

2) The identification of peripheral tissues or cell lines easily accessible at a non invasive level which could be used for the diagnostic and therapeutic adjustment.

In the present invention the validation of this strategy was obtained by:

A - The measurement of adequate markers of the editing process to all or part of the implicated editing regulation: 5-HT2cR mRNA editing profiles, markers of the expression of the different isoforms of the editing enzymes: ADAR1-150, ADAR1-110, ADAR2.

As an example of the results obtained to validate the present invention the table I precises the set of markers which are proposed after their identification in the proposed sources from Human subjects and experimental animals or cell lines:

Table 1: Level of expression of SHT2cR and editing enzymes. We note that in the blood samples the level of expression of the editing enzymes can be easily determined. In skin samples the 5-HT2cR is expressed and is submitted to the editing activity of ADARs 1 and 2 and can complete the investigation of the general state of regulation of the editing of this receptor in the brain samples.

| | | Brain | Skin | Blood |
|---|---|---|---|---|
| 5-HT2cR | | | | |
| Mouse (Balb/c) | | +++ | + Edited by ADARS1 and 2 | 0 |
| Human | | +++ | + Edited by ADARS1 and 2 | 0 |
| | | | | |
| ADARs | | | | |
| Mouse | ADAR1-150 | ++ | +++ | ++ |
| | ADAR1-110 | ++ | +++ | + |
| | | | | |
| | ADAR2 | ++ | ++++ | ++ |
| | | | | |
| Human | ADAR1-150 | ++ | ** | ++ |
| | ADAR1-110 | ++ | ** | ** |
| | | | | |
| | | | | |
| | ADAR2 | + | ** | ** |

For each marker the samples are rapidly extracted with special care for allowing the possibility to execute the determination of the levels of expression of mRNA, the editing profiles and the determination of proteins markers by western blotting from the same tissue or cell sample.

The table 2 summarizes the preferred processes used for the determination of level of expression of the used biomarkers.

Table 2

| | 5-HT2cR (mouse) | 5-HT2cR (Hum) | ADAR1 p110 (mouse) | ADAR1 p150 (mouse) | ADAR2 (mouse) | ADAR1 p110 (Hum) | ADAR1 p150 (Hum) | ADAR2 (Hum) |
|---|---|---|---|---|---|---|---|---|
| RNA | polyA+ or Total | polyA+ or Total | Total | Total | Total | Total | Total | Total |
| DNAase digestion | Yes | Yes | Yes | Yes | Yes | Yes | Yes | Yes |
| RT primer | 5-HT2cR-oligo6-RT | 5-HT2cR-oligo6-RT | oligo(dT)20 | oligo(dT)20 | oligo(dT)20 | oligo(dT)20 | oligo(dT)20 | oligo(dT)20 |
| RNAaseH treatment | Yes | Yes | Yes | Yes | Yes | Yes | Yes | Yes |
| PCR1 | PCR9/PCR10 | PCR9/PCR10 | EX1B 72p/EX2 646m | EX1A 19p/EX2 646m | EX7 1281p/EX9 1622m | EX1B 534p/EX2 804m | EX1A 34p/EX2 578m | ADAR2 1274p/ADAR21486m |
| PCR2 | PCR1/PCR4 | PCR2/PCR18 | | | | | | |

The following examples illustrate typical procedure and results.

**EXAMPLE 2:** Obtention of the complete editing profile from one sample of brain tissue (figure 1)

Total RNA was extracted and purified from tissue or cell extracts, according to manufacturer's specifications (Qiagen RNeasy, Mini Kit). The quantity and purity of the extracted RNA were assessed by measuring both the absorbance at 260nm and the 260/280 nm ratio with a GeneQuant spectrophotometer (PharmaciaBiotech). In order to eliminate possible contamination by genomic DNA, 8 $\mu$l of each RNA (between 88 ng and 1.3 $\mu$g) were then treated with 1 unit of DNase I (Invitrogen) for 15 min at room temperature in a final volume of 10 $\mu$l. The reaction was stopped by adding 1 $\mu$l of 25mM EDTA and then heated for 10 min at 65°C. The reverse transcription of DNAse I-treated RNAs (10 $\mu$l) was performed using 15 units of ThermoScript reverse transcriptase (ThermoScript RT-PCR System, Invitrogen) and Oligo(dT) primers at a final concentration of 2.5 $\mu$M.

A first PCR reaction (final volume 25 $\mu$l) resulting in a 250 bp fragment, was then carried out on 1 $\mu$l of the reverse transcription products with 0.2 unit of Platinum *Taq* DNA polymerase (ThermoScript RT-PCR system, Invitrogen) and specific primers (forward primer: 5'-TGTCCCTAGCCATTGCTGATATGC-3' (SEQ ID No. 35) and reverse primer: 5'-GCAATCTTCATGATGGCCTTAGTC-3' (SEQ ID No. 36); final concentration of each 0.2$\mu$M) located on exon IV and exon V of the Human 5-HT2cR cDNA, respectively. After a denaturing step at 95°C for 3 min, the PCR was brought to its final point after 35 cycles (15s at 95°C; 30s at 60°C; 20s at 72°C), and a final elongation step of 2 min at 72°C. Aliquots of the amplification products were used to check the product on a 2% agarose analytic gel.

**Second PCR and separation of single-strand cDNA fragments by Capillary Electrophoresis (CE)**

1 $\mu$l of a 1/50 dilution of the RT- 1st PCR products, or the 250 bp cDNA amplified from plasmids harboring the thirty-two standard of human 5-HT2cR (or 5HT2CR) isoforms, were used as templates for an additive nested-PCR. These 32 standards, corresponding to the non-edited (NE) and edited isoforms of human 5-HT2cR. Amplifications were performed in a final volume of 20 $\mu$l with HPLC-purified fluorescent primers (forward primer: FAM-ATGTGCTATTTTCAACAGCGTCCATC-3' (SEQ ID No. 37); reverse primer: VIC-GCAATCTTCATGATGGCCTTA-3' (SEQ ID No. 38); final concentration of each 0.2 $\mu$M), and 0.2 unit of Platinum *Pfx* DNA polymerase (Invitrogen).

The VIC-labelled reverse primer hybridizes to a complementary sequence of the 5-HT2c receptor identical in human, mouse and rat. On the other hand, although used with human samples, the sequence of the FAM-labelled foward primer was designed to be as close as possible to that of the mouse. More precisely, T residues in positions 5 and 6 of the human oligonucleotide sequence (positions 1133 and 1134 of human reference U49516) were changed into G and C, respectively.

Simulations of stochastic folding pathways of both strands of the PCR product obtained with the two primers described above were carried out with the Kinefold server (kinefold.curie.fr). They showed that the lowest free-energy structures obtained for forward and reverse strands - the edited region embedded in the loop of a stem-loop structure, and able to hybridize with a complementary sequence located elsewhere in the whole structure after folding of the stem - were very close to that calculated for a mouse nested-PCR product successfully used for Mouse samples. This set of primers was shown to be optimal for conformational analysis of human 5HTR2C mRNA editing by non denaturing capillary electrophoresis by single strand conformational polymorphism (CE-SSCP).

The amplified fragment is 127 bp-long. As for RT-PCR, after an initial denaturing step of 5 min at 94°C, the amplification reaction was brought to an end with 35 cycles (15s at 94°C; 30s at 55°C; 20s at 68°C) and a final elongation step of 2 min at 68°C. Again, quality of the 127 bp-long amplified fragments were assessed on a 2% agarose gel before subsequent analysis in a 3100 Avant Genetic Analyser (Applied Biosystem).

Fluorescent PCR products corresponding to standard isoforms (1 $\mu$l of a 1/100 dilution in DEPC treated water) and samples (1 $\mu$l of a 1/30 dilution) diluted in 11 $\mu$l of deionized formamide were added to a mixture of ROX labelled migration standards (MWG-BIOTECH, AG) (0.5 $\mu$l each) covering the whole range of the electrophoregram retention times. These ROX standards were used for CE calibration and subsequently to obtain correct superimposition of standards and samples peaks. After denaturing for 2 min at 95°C, samples were immediately chilled on ice. Non-denaturing CE was carried out in an ABI PRISM 3100-*Avant* Genetic Analyser (Applied Biosystems) through 80 cm-long capillaries filled with 7% "POP Conformational Analysis Polymer" (Applied Biosystems), 1X TBE and without glycerol. After a prerun performed at 15 kV for 3 min, samples were injected for 15s at 2 kV, and electrophoresis was run for 105 min at 15 kV at a controlled temperature of 20°C. Under these conditions, each of the thirty-two possible isoforms were clearly resolved as a result of the single ssDNA conformation obtained with either the FAM-labelled or the VIC-labelled strand. The different retention times were used for unambiguous identification of the isoforms.

**Identification and relative quantification of each isoform in each brain sample**

The Electrophoretic Signal was then processed using an in-house software. First, the time basis of electrophoretic profiles of each sample was adjusted using the ROX-labelled strands of the migration standards. This allowed FAM- and VIC-labeled strands to precisely deconvolute the standards and samples signals in a unique time basis. Background was then adjusted and subtracted and then total area under each signal normalized.

The relative proportion of each isoform was processed by a best fitting of each deconvoluted and normalized analytical signal of the brain samples. It was performed by the iterative adjustment of the integrated signal represented by the 32 similarly deconvoluted and normalized standard analytical signals. The calculation was based on the hypothesis that the SSCP signal $S(t) = \sum_{i=1}^{N} g_i R_i(t)$ in which $R_i(t)$, with $i \in \{1,.., N\}$, are the standard signals and $g_i$ the % of each of them in the signal. The best fit minimized the sum of squares due to error (SSE) $SSE = \int \left[ S(t) - \sum_{i=1}^{N} g_i R_i(t) \right]^2$ and was controlled by the least square statistical analysis.

The result of this best fitting was statistically evaluated after calculation of the r² value such as $r^2 = 1 - \frac{SSE}{SSM}$ in which SSM is the Sum of Square about Mean such as $SSM = \sum_{i=1}^{t} (S(t) - \overline{S})^2$. The maximum theoretical best fit would give an r² = 1.

All experiments were carried out under blind conditions and all samples were assayed in the same batch for RT-PCR and second PCR reactions. The best fitting results yielded a specific editing profile for each individual sample, which was determined by the percentage of each edited and non edited form of the total analytical signal. These initial values were used for statistical analyses.

This method gives the proportion of each expressed mRNA isoform expressed as the percentage of the total of 5-HT2c receptor present in the extract.

As an example is given here the table of identification of the 32 human isoforms in which each FAM and VIC labelled strands gives a set of retention time (see figure 1). It is easy to note that the use of the two strands can solve the total identification of the 32 standards isoforms.

The main advantage of this processing is to give a complete quantitative estimation of the distribution of the expressed of SHT2cR isoforms (editing profile) in a given concentration of 5HT2cR mRNA. This is obtained from one single assay and allows to easily determine the characteristics of this profile in a given situation. With this technique it has been possible to demonstrate in a group of 6 depressed patients having committed suicide a specific signature which characterized the depressed group of patients. This signature gives interesting information about the dysregulation of editing process occurring in the dorsal prefrontal regions of the brain and strongly support the interest to explore the steady state of editing enzymes in skin and blood samples of depressed patients.

**EXAMPLE 3:** 5HT2CR expression in Human and Mouse skin

Skin, because the dermal presence of 5-HT2c receptors and editing enzymes could be an interesting source for measuring at the periphery both the 5-HT2cR editing and the level of expression of the editing enzymes.

Figure 2 represents a typical control of RTPCRs performed after extraction of polyA+ RNAs from Human (A) or Mouse (B) skin samples.

The application of the capillary electrophoresis separation of the SSCP products of the second nested PCR products led to achieve the demonstration that, in the human and Mouse skin, the ADAR1 and ADAR2 editing enzymes were active and that pathological or physiopathological states could modify in this peripheral tissue the editing regulation of the 5-HT2cR.

**EXAMPLE 4:** Location and nature of the expression of ADAR1 and ADAR 2 isoforms

As predicted by the preceding validation experiments, the expression of ADAR1 and ADAR 2 isoforms were found expressed in the skin samples of Mouse and Human Skin.

The figure 3 shows an example of control of the RT/PCR identification of ADAR1 150, ADAR 1 110 and ADAR 2 in experiments performed after extraction of Mouse total blood or skin RNAs.

In Man it was also possible to identify and to easily quantify the level of expression of editing enzymes after collection of a small volume of blood.

With a typical yield of 1-2 x $10^6$ leukocytes per ml of freshly collected blood, a volume of 5 ml allows to isolate enough total RNA for reverse transcription reactions. Blood samples (5ml) are collected into heparinized tubes. The following steps of the protocol must be immediately carried out under sterile conditions. Dilute the anticoagulated sample material with an equal volume of 0.9% NaCl sterile solution, or 1X PBS sterile solution, or RPMI 1640 sterile culture medium. Separation medium (eg Ficoll-Paque Plus, GE Healthcare Bio-Sciences AB, ref.: 17-1440-02 or 17-1440-03) must be warmed-up to room temperature just before use and protected from light. Fill 15 ml LeucoSep tube (Greiner Bio-One, ref.: 163 289 or 163 290) with 3 ml of separation medium. Centrifugate for 30 s at 1000 g and room temperature (the separation medium is then below the porous barrier of the tube). When using LeucoSep tubes that are pre-filled with separation medium, the aforementioned steps can be cancelled (ref.: 163 288 or 227 288). Simply warm-up the tubes to RT. Pour carefully the anticoagulated, diluted material sample (1:2 in balanced salt solution or RPMI 1640, see above) into the 15 ml LeucoSep tube. Centrifugate 10 minutes at 1000 g and room temperature, or 15 minutes at 800 g and room temperature in a swinging bucket rotor.

Switch-off brakes of the centrifuge

After centrifugation, harvest the enriched cell fraction (lymphocytes/PBMCs = white ring) by means of a Pasteur pipette. Wash the enriched cell fraction with 10 ml of 1X PBS sterile solution, subsequently centrifugate 10 minutes at 250 g. Repeat washing step twice. For the last centrifugation, pellets must be collected into microcentrifuge tubes (1.5 ml Eppendorf tubes) via resuspension in 1 ml 1X PBS sterile solution. After the last centrifugation (10 minutes at 250 g and room temperature) discard the supernatant and quickly cover the "dryed" pellets of enriched cell fractions with RNAlater RNA stabilization Reagent (Qiagen, ref.: 76104 or 76106). If the submerged pellets are stored at 2-8°C, the lymphocytes/PBMCs gene expresion profile can be stabilized up to 4 weeks at this temperature. If transporting samples in RNA*later* reagent, ensure that the pellets always remain submerged in the reagent. Either keep the tubes upright during transport or fill the tubes completely with the stabilization solution. During transport tubes can be kept in a polystyrene box filled with blue ice packs. A better solution could be to directly lyse the pellets of the enriched cell fraction in 1 ml of TRIzol Reagent (Invitrogen, ref.: 15596-026), kept and sent at room temperature. As this lysis reagent contains phenol, samples tubes must be tightly closed. Actually, as described by the furnisher (Invitrogen), the leukocytes lysate in TRIzol reagent (see above) allows later extractions of both total RNA (aqueous phase) and proteins (organic phase). Tubes could be sent at room temperature or in dry ice.

An example of validation is given on figure 4 in which is presented the control of the RTPCR products of editing enzymes in human brain, skin and CD4 and CD8 blood cells, samples.

It is thus possible, in Human, to correctly analyse the expression of the editing enzymes in Brain (post mortem studies), Skin and Blood samples (diagnostic studies) as illustrated on the control experiment presented on figure 5.

All the elements of the table 2 being verified, it becomes possible to elucidate the precise conditions and the limits of the analysis of blood and skin editing enzyme expression and 5-HT2/C editing profile in skin as biomarkers in diagnostic and treatment of several pathological states in human with a deep validation coming from adequate physio- or pharmaco-pathological models.

**EXAMPLE 5:** Analysis of the isoforms of the 5-HT2c receptor in post mortem suicide-depressed patients brain samples compared with patient controls samples

The analytical process illustrated in figure 1 allows analysis of all isoforms of the 5-HT2c receptor in post mortem brain samples. A study of 6 patient controls and carefully-selected suicide-depressed patients showed that: (1) specific brain regions show a specific pattern of distribution of the edited and non-edited 5-HT2CR isoforms, and (2) this distribution pattern is significantly altered in the human anterior cingulate cortex and dorsal prefrontal cortex, which are involved in the pathophysiology of major depression. These changes in the isoform signature are illustrated in table 3, which shows the effects observed in the anterior cingulate cortex. Importantly, this technique can measure dynamic changes in isoform prevalence in both directions-increases or decreases compared to controls, providing insight into the potential enzyme dysfunction underlying the changes. Here, for example, ADAR I activity (which specifically edits the A and B sites and, with less specificity, the C site) is up-regulated.

Table 3: Editing profiles obtained in controls and suicide depressed patients. The total editing profile was measured in patients from both groups (n=6 in each). Results represent the isoform prevalence as a percent of all isoforms and given as the mean $\pm$ SEM. The two aspects of the signature-the distribution of all isoforms and the distribution according to individual editing sites-were analyzed by ANOVA II.

Table 3

| Anterior Cingulate Cortex | | | | | | | | | | | | | | | | | | | | | | | | | | | |
|---|---|---|---|---|---|---|---|---|---|---|---|---|---|---|---|---|---|---|---|---|---|---|---|---|---|---|---|
| **Distribution of mRNA Isoforms** | | | | | | | | | | | | | | | | | | | | | | | | | | | |
| mRNA Isoforms | AE | ABDE | BCE | CE | D | ABE | AC | AD | ACD | BCD | ACDE | ADE | ABCD | BDE | B | C | NE | DE | BD | CD | A | AB | ACE | ABD | BC | ABC | ABCE | ABCDE |
| Corresponding Proteins | VDI | VDV | MGI | IGI | INV | VDI | VSI | VNV | VSV | MSV | VGV | VDV | VSV | MDV | MNI | ISI | INI | IGV | MNV | ISV | VNI | VNI | VGI | VNV | MSI | VSI | VGI | VGV |
| Mean (Controls) | 1.0 | 2.2 | 0.1 | 1.6 | 3.7 | 1.4 | 4.0 | 6.8 | 5.2 | 0.4 | 2.5 | 0.6 | 16.3 | 0.3 | 0.7 | 3.9 | 8.1 | 0.9 | 1.1 | 1.4 | 6.5 | 5.0 | 4.1 | 10.6 | 0.8 | 5.9 | 0.7 | 1.8 |
| SEM | 0.5 | 0.4 | 0.1 | 0.5 | 0.4 | 0.4 | 0.5 | 1.3 | 1.1 | 0.1 | 0.5 | 0.1 | 2.2 | 0.3 | 0.3 | 0.4 | 1.6 | 0.1 | 0.3 | 0.3 | 0.8 | 1.0 | 0.8 | 1.0 | 0.2 | 0.9 | 0.2 | 0.7 |
| Mean (Depressed) | 0.3 | 0.9 | 0.1 | 0.7 | 1.7 | 0.6 | 3.2 | 5.5 | 4.3 | 0.3 | 2.3 | 0.5 | 15.1 | 0.3 | 0.7 | 4.1 | 8.9 | 1.0 | 1.2 | 1.6 | 7.5 | 6.6 | 5.5 | 14.9 | 1.2 | 10.1 | 1.3 | 4.0 |
| sem | 0.1 | 0.2 | 0.0 | 0.2 | 0.5 | 0.1 | 1.0 | 0.7 | 1.0 | 0.2 | 0.6 | 0.1 | 3.4 | 0.1 | 0.3 | 0.7 | 1.4 | 0.0 | 0.6 | 0.4 | 0.7 | 0.1 | 1.7 | 2.1 | 0.3 | 1.6 | 0.4 | 0.7 |
| % of variation versus Control | -75.9 | -62.0 | -59.0 | -56.2 | -55.0 | -54.3 | -19.8 | -19.2 | -17.7 | -17.6 | -9.8 | -9.7 | -7.1 | 0.0 | 1.9 | 4.6 | 10.8 | 11.3 | 12.9 | 13.1 | 15.1 | 30.4 | 35.4 | 40.1 | 47.1 | 70.7 | 75.9 | 121.8 |
| p values (t test) | 0.091 | 0.004 | 0.155 | 0.062 | 0.004 | 0.035 | 0.256 | 0.195 | 0.279 | 0.398 | 0.373 | 0.371 | 0.391 | 0.500 | 0.488 | 0.412 | 0.345 | 0.158 | 0.418 | 0.353 | 0.187 | 0.071 | 0.228 | 0.049 | 0.157 | 0.021 | 0.106 | 0.038 |
| p ANOVA factor: depressed group | p=0.03 | | | | | | | | | | | | | p=0.0003 | | | | | | | | | | | | | | |

**Distribution of edited sites in the structure**

| | A | B | C | D | E |
|---|---|---|---|---|---|
| Mean Controls | 74.9 | 47.2 | 47.3 | 49.9 | 15.4 |
| SEM | 1.8 | 2.9 | 3.2 | 1.7 | 1.9 |
| Mean Depressed | 82.6 | 57.0 | 53.1 | 51.6 | 16.4 |
| SEM | 0.8 | 0.4 | 0.5 | 0.4 | 0.3 |
| % of variation versus Control | 10.3 | 20.8 | 12.4 | 3.4 | 6.1 |
| p values (t test) | 0.04 | 0.01 | 0.09 | 0.24 | 0.35 |
| p values ANOVA | 0.030 | | 0.600 | | |
| Factor: depressed group | 0.001 | | | | |

**EXAMPLE 6:** Interferon alpha2 treatment in mouse induces significant increase in ADAR1 expression in blood. This peripheral effect is also identified in the skin and in brain.

This experiment was performed in Balb/cJ Mouse. A dose of 20,000 IU of mouse alpha interferon was injected by IP route and mice groups (n=8) killed at time 3, 6 and 8 hours after injection. A control group (n=8) was killed at time zero. The blood, skin and brain were rapidly processed to avoid RNA degradation. Total RNA was extracted from each tissue sample and specific mRNA coding for inducible ADAR1 was quantified by QPCR using GAPDH endogenous gene as reference. The results are summarized on figure 6. They clearly show that when a significant increase in ADAR 1 expression is observed in blood samples of interferon treated mice, an amplified response is also observed in skin samples and in prefrontal cortex.

Additionally, the editing profile of the 5-HT2cR was determined according to the methods previously described (see example 2) in the prefrontal area of control and interferon treated mice killed at 8 hours. In table 4 it is easy to see that:

1) Following the induction of the ADAR 1 expression occurring rapidly in brain, in these experimental conditions, a significant early alteration of the editing process of the 5-HT2cR is seen at 8 Hours. A group of 12 edited isoforms including the ABCD and ACD isoforms was found significantly increased. They represent more than 30 % of the total 5-HT2cR mRNA.

2) The analysis of the proportions of edited sites in this group clearly exhibit a significant increase in the proportions of A, B, and C sites found edited. They can be interpreted as mainly resulting from a greater activity of ADAR1. This is confirmed when the analysis is restricted to the isoforms of this group which are exclusively due to the activity of ADAR 1.

Table 4: Analysis of the alteration of the editing profile of 5-HT2cR mRNA after interferon treatment. In each individual, the total profile of editing was measured as the proportion of each edited isoforms in the total specific mRNA of the receptor. Presented results are the mean ± SEM of controls and interferon treated mice killed at 8 Hours after injection. P values were calculated from Student test. Are thus presented : the sum of the edited isoforms found increased in the interferon treated goup, the proportion of the A,B,C,D and E sites found edited in this group of isoforms and the relative proportion of mRNA represented by the isoforms of this group which is exclusively the result of a specific action of ADAR1.

Finally it becomes reasonable to propose that the measure of changes in ADARs expression at the periphery (blood) could predict important alteration of editing in the brain which could explain the secondary effects on mood of several already used treatments in several therapeutic fields.

| Increased edited 5-HT2c R mRNA isoforms | | | | | | | | | | | | |
|---|---|---|---|---|---|---|---|---|---|---|---|---|
| ABCDE (VGV) | ABC (VSI) | CDE (IGV) | **AVBCD (VSV)** | ABCE (VGI) | D (INV) | **ACD (VSV)** | ABE (VDI) | BD (MNV) | AC (VSI) | BCDE (MGV) | B (MNI) | E (IDI) |

| represents (% of total) | | | |
|---|---|---|---|
| controls | INF treated | % of variation | p |
| 29.6 ± 0.6 | 32.1 ± 0.4 | 10.1 | **0.005** |

Corresponding proportion of sites found edited

| | controls | INF treated | % of variation | p | |
|---|---|---|---|---|---|
| **A** | **23.8 ± 0.6** | **25.3 ± 0.3** | **6.1** | **0.03** | |
| **B** | **22.6 ± 0.6** | **24.2 ± 0.3** | **6.9** | **0.02** | **ADAR1** |
| **C** | **23.8 ± 0.6** | **25.3 ± 0.3** | **6.4** | **0.03** | |
| E | 3.2 ± 0.2 | 3.6 ± 0.2 | 13.7 | 0.06 | ADAR2 |
| D | 22.4 ± 0.8 | 24.0 ± 0.4 | 6.7 | 0.09 | |

Increased Isoforms exclusively due to ADAR1 action

| **ABC (VSI)** | **ABCE (VGI)** | **ABE (VDI)** | **AC (VSI)** | **B (MNI)** | |
|---|---|---|---|---|---|
| represents (% of total) | | | | | |
| controls | INF treated | % of variation | p | **ADAR1** | |
| **7 ± 0.3** | **7.7 ± 0.2** | **10.1** | **0.03** | | |

**Claims**

1. A method implementing a biological sample consisting of a peripherical tissue containing cells for evaluating the pathological alteration of the ADAR dependent A to I mRNA editing of the 5HTR2C in the brain, wherein said peripherical tissue containing cells is a mammal skin sample selected from the group consisting of skin cells line, cultured skin-derived cells and skin tissue sample, and wherein the pathological alteration of the ADAR dependant A to I mRNA editing of the 5HTR2C in the brain is detected by measuring 5HTR2C editing in the skin sample.

2. The method according to claim 1, wherein said pathology is selected from the group consisting of mental disorders, schizophrenia, depression, depressed suicide or abnormal feeding behaviour.

3. The method according to claim 1 or 2, wherein this method further comprises the step of determining the ADARs expression products contained in a second peripherical tissue sample which is a blood sample containing cells of said patient or mammal.

4. The method according to claim 3, wherein the blood sample cells expressing ADARs are blood white cells or leucocytes.

5. The method according to any one of claims 1 to 4, wherein said editing rate or said profile of the edited forms is determined for all the edited and unedited forms of said 5HTR2C mRNA.

6. The method according to any one of claims 1 to 5, wherein the editing rate for each edited and unedited form or the profile of the edited forms of said 5HTR2C mRNA is determined by a method which comprises the following steps:

   A) extraction of the total RNAs of said skin cells line, cultured skin-derived cells or skin tissue, followed, where appropriate, by purification of the mRNAs;
   B) reverse transcription of the RNAs extracted in step A); and
   C) PCR amplification of the cDNAs obtained in step B) using at least a pair of primers specific for the 5HTR2C mRNA fragment containing the edition sites which may be edited, this pair of primers being chosen so as to be able to amplify all the editing forms and the unedited form potentially present in the RNA extract,
   and

      - wherein the step B) of reverse transcription is carried out by using an oligonucleotidic primer specific of the 5HTR2C gene having the sequence SEQ ID No. 34;
      - wherein in step C), the PCR amplification step is a nested type PCR comprising two rounds of PCR, and wherein the first round of PCR is carried out by a set of primers which results to a PCR nucleic acid product having a length comprised between 200 bp and 300 bp, and wherein the second round of PCR is carried out by a set of primers which results to a final PCR nucleic acid product having a length comprised between 90 bp and 160 bp.

7. The method according to claim 6, wherein in step C), the PCR amplification step is a nested type PCR comprising two rounds of PCR,

   - wherein, for mouse, rat or human, the first round of PCR is carried out by the set of primers:

      forward primer 5'-TGTCCCTAGCCATTGCTGATATGC-3', and
      reverse primer 5'-GCAATCTTCATGATGGCCTTAGTC-3'; and

   - wherein for human, the second round of PCR is carried out by the following sets of primers:

      forward primer FAM-ATGTGCTATTTTCAACAGCGTCCATC-3', and
      reverse primer VIC-GCAATCTTCATGATGGCCTTA-3'; and

   - wherein for mouse and rat, the second round of PCR is carried out by the following set of primers:

      forward primer 5'-TTTGTGCCCCGTCTGGAT-3', and
      reverse primer 5'-GCCTTAGTCCGCGAATTG-3'.

8. The method according to claim 7, wherein the editing rate for each edited and unedited form or the profil of the edited forms of said 5HTR2C mRNA is determined by an SSCP method capable of providing the editing profile for each of the edited and unedited separate forms of said mRNA, said SSCP method being **characterized in that** it comprises after the steps A), B) and C) the following steps:

   D) where appropriate, purification of the PCR products obtained in step C);
   E) where appropriate, quantification of the PCR products obtained in step D);
   F) dissociation of the double-stranded cDNAs to single-stranded cDNAs, in particular by heating followed by abrupt cooling;
   G) separation of the single-stranded cDNAs by capillary electrophoresis; and
   H) obtaining of the editing profile by reading of the fluorescence and, where appropriate, acquisition of the profile data by means of the exploitation system associated with the fluorescence reader.

9. The method according to any one of claims 1 to 8, wherein the ADAR expression products are ADAR1, isoforms 150 and/or 110, and the ADAR2 gene expression products, preferably the expression products of the human gene encoding the ADAR1, isoforms 150-kD and/or 110-kD protein, and the ADAR2 protein.

10. The method according to any one of claims 1 to 9, wherein the ADAR expression products are the ADAR mRNAs.

11. A method according to claim 10, wherein), the determination of the ADAR mRNA is carried out by a method which comprises the following steps:

   A) extraction of the total RNAs of said sample cells, followed, where appropriate, by purification of the mRNAs;
   B) reverse transcription of the RNAs extracted in step A); and
   C) PCR amplification of the cDNAs obtained in step B) using at least a pair of primers specific for each of the ADAR mRNA to be quantified and/or qualitatively analysed, and wherein in step C), the pair of primers specific for the ADAR mRNA PCR amplification are selected from the group consisting of:

   - for human ADAR1-150 isoform mRNA amplification:

      Forward: 5'-GCCTCGCGGGCGCAATGAATCC-3',
      Reverse: 5'-CTTGCCCTTCTTTGCCAGGGAG-3';

   - for human ADAR1-110 isoform mRNA amplification:

      Forward: 5'-CGAGCCATCATGGAGATGCCCTCC-3',
      Reverse: 5'-CATAGCTGCATCCTGCTTGGCCAC-3';

   - for human ADAR2 mRNA amplification:

      Forward: 5'-GCTGCGCAGTCTGCCCTGGCCGC-3',
      Reverse: 5'-GTCATGACGACTCCAGCCAGCAC-3';

   - for mouse ADAR1-150 isoform mRNA amplification:

      Forward: 5'-GTCTCAAGGGTTCAGGGGACCC-3',
      Reverse: 5'-CTCCTCTAGGGAATTCCTGGATAC-3';

   - for mouse ADAR1-110 isoform mRNA amplification:

      Forward: 5'-TCACGAGTGGGCAGCGTCCGAGG-3',
      Reverse: 5'-CTCCTCTAGGGAATTCCTGGATAC-3'; and

   - for mouse ADAR2 mRNA amplification:

      Forward: 5'-GCTGCACAGTCTGCCTTGGCTAC-3',
      Reverse: 5'-GCATAAAGAAACCTGAGCAGGGAC-3'.

**12.** A method according to any one of claims 1 to 9, wherein the ADAR expression products are the ADAR proteins.

**13.** Use of a method according to any one of claim 1 to 12, for identifying *in vitro* whether a patient presents a pathology or is at risk to develop a pathology related to an alteration of the mechanism of the mRNA editing in the brain of the 5HTR2C, from a biological sample of the patient to be tested containing skin cells, wherein this method comprising the following steps of:

a) determining the editing rate or profil of the edited forms of said 5HTR2C mRNA expressed in said sample of skin cells; and, optionally
b) determining the nature or/and the quantity of the ADARs expressed in said sample of skin cells; and
c) identifying whether said patient presents or is at risk to develop such a pathology by comparing the editing rate or profil of said 5HTR2C mRNA and the nature or/and the quantity of the ADARs expressed in said sample obtained in step a) and b) with characteristic control editing rate or profil of the 5HTR2C mRNA and expressed ADARs profil obtained for normal patients or for patients exhibiting pathologies related to an alteration of the mechanism of this mRNA editing in the brain.

**14.** Use of a method according to any one of claim 1 to 12, for determining *in vitro* whether a pathology exhibited by a patient is related to an alteration of the mechanism of the mRNA editing in the brain of the 5HTR2C, from a biological sample of the patient to be tested containing skin cells, wherein this method comprising the following steps of:

a) determining the editing rate or profil of the edited forms of said 5HTR2C mRNA expressed in said sample of skin cells; and, optionally
b) determining the nature or/and the quantity of the ADARs expressed in said sample of skin cells; and
c) identifying whether said patient presents or is at risk to develop such a pathology by comparing the editing rate or profil of said 5HTR2C mRNA and the nature or/and the quantity of the ADARs expressed in said sample obtained in step a) and b) with characteristic control editing rate or profil of the 5HTR2C mRNA and expressed ADARs profil obtained for normal patients or for patients exhibiting pathologies known to be not related to an alteration of the mechanism of this mRNA editing.

**15.** Use of a method according to any one of claims 1 to 12, for identifying in *vitro* an agent that modulates *in vivo* the editing of the 5HTR2C mRNA in the brain of a mammal, from a biological sample of said mammal containing skin cells, said candidate modulator of the 5HTR2C mRNA editing being beforehand administered to said mammal, said method comprising the following steps of:

a) determining the effects of said modulator

- on the editing rate or profil of the edited forms of said 5HTR2C mRNA expressed in said sample of skin cells; and, optionally
- on the nature or/and the quantity of the ADARs expressed in said sample of skin cells, by comparing the editing rate or profil of said 5HTR2C mRNA and the nature or/and the quantity of the ADARs expressed in said sample obtained in step a) with the editing rate or profil of said 5HTR2C mRNA and the nature or/and the quantity of the ADARs expressed obtained from control sample of skin cells.

**16.** Use of a method according to any one of claim 1 to 12, for determining *in vitro* the efficiency of a drug used for the prevention or for the treatment in a patient of a pathology related to an alteration of the mechanism of the mRNA editing in the brain of the 5HTR2C, from a biological sample of the patient containing skin cells obtained before and during or/and after the treatment, wherein this method comprising the following steps of:

a) determining in said samples containing skin cells obtained before and during or/and after the treatment:

- the editing rate or profil of the edited forms of said 5HTR2C mRNA expressed in said samples of skin cells; and, optionally
- the nature or/and the quantity of the ADARs expressed in said samples of skin cells; and

b) determining the efficiency of said drug by comparing the editing rate or profil of said 5HTR2C mRNA and the nature or/and the quantity of the ADARs expressed in said samples obtained in step a), a modulation of the editing rate or profil and the nature or/and the quantity of the ADARs expressed resulting to an editing rate or profil and a nature or/and a quantity of the ADARs expressed close or equal to this observed for normal patients being significant of the efficiency of the treatment.

**17.** Use of a method according to any one of claims 1 to 12, for determining if a patient responds or does not respond to a treatment of a pathology resulting or provoking by the alteration of the mechanism of the mRNA editing in the brain of the 5HTR2C, further comprising a steps of:

c) determining if the patient responds or not responds to the treatment by observing the modification of the editing rate or profile and, optionally, the nature or/and the quantity of the ADARs expressed after a period of treatment (i.e. 15 days, 30 days, 2 months, 6 months, etc.) by comparing with the editing rate or profile and, optionally, the nature or/and the quantity of the ADARs expressed before the beginning of the treatment.

**18.** Use of a method according to any one of claims 1 to 12, for identifying *in vitro* an agent that modulates *in vitro* the editing of the 5HTR2C mRNA in a mammal, said method comprising the following steps of:

a) obtaining a biological sample containing cells from mammalian skin cells line, optionally, these cells can be recombinant cells;
b) contacting said biological sample in the presence of a candidate modulator of said 5HTR2C mRNA editing; and, optionally
c) determining the effects of said modulator

- on the editing rate or profil of the edited forms of said 5HTR2C mRNA expressed in said sample of skin cells; and
- on the nature or/and the quantity of the ADARs expressed in said sample of skin cells, by comparing the editing rate or profil of said 5HTR2C mRNA and the nature or/and the quantity of the ADARs expressed in said sample obtained in step c) with the editing rate or profil of said 5HTR2C mRNA and the nature or/and the quantity of the ADARs expressed obtained from control sample of skin cells.

**Patentansprüche**

**1.** Verfahren zum Implementieren einer biologischen Probe bestehend aus einem zellhaltigen peripheren Gewebe zum Evaluieren der pathologischen Veränderung des ADAR-abhängigen A-zu-I-mRNA-Editings des 5HTR2C im Gehirn, wobei das zellhaltige periphere Gewebe eine säugetier Hautprobe ist, die ausgewählt ist aus der Gruppe bestehend aus einer Hautzelllinie, kultivierten von Haut abgeleiteten Zellen und einer Hautgewebeprobe, und wobei die pathologische Veränderung des ADAR-abhängigen A-zu-I-mRNA-Editings des 5HTR2C im Gehirn durch ein Bestimmen des 5HTR2C-Editings in der Hautprobe nachgewiesen wird.

**2.** Verfahren nach Anspruch 1, wobei die Pathologie ausgewählt ist aus der Gruppe bestehend aus bestehend aus psychischen Störungen, Schizophrenie, Depression, depressivem Suizid oder abnormalem Essverhalten.

**3.** Verfahren nach Anspruch 1 oder 2, wobei das Verfahren ferner den Schritt des Bestimmens der ADAR-Expressionsprodukte umfasst, die in einer zweiten peripheren Gewebeprobe enthalten ist, welche eine Blutprobe enthaltend Zellen des Patienten oder Säugers ist.

**4.** Verfahren nach Anspruch 3, wobei die ADAR-exprimierenden Blutprobenzellen weiße Blutzellen oder Leukozyten sind.

**5.** Verfahren nach einem der Ansprüche 1 bis 4, wobei die Editingrate oder das - profil der Editingformen für alle editierten oder uneditierten Formen der 5HTR2C-mRNA bestimmt wird.

**6.** Verfahren nach einem der Ansprüche 1 bis 5, wobei die Editingrate für jede editierte und uneditierte Form oder das Profil der editierten Formen der 5HTR2C-mRNA durch ein Verfahren umfassend die folgenden Schritte bestimmt wird:

A) Extrahieren der Gesamt-RNA der Hautzelllinie, der kultivierten, von Haut abgeleiteten Zellen oder des Hautgewebes, gefolgt von, falls geeignet, einer Aufreinigung der mRNAs;
B) reverse Transkription der in Schritt A) extrahierten RNAs; und
C) PCR-Amplifikation der in Schritt B) erhaltenen cDNAs unter Verwendung wenigstens eines Paars von Primern, die für das 5HTR2C-mRNA spezifisch sind, welche die Editingstellen enthält, die editiert werden können, wobei das Primerpaar so ausgewählt ist, dass es alle editierten Formen und uneditierten Formen amplifizieren kann, die potentiell im RNA-Extrakt vorliegen, und

- wobei Schritt B) der reversen Trankription unter Verwendung eines für 5HTR2C spezifischen Oligonukleotidprimers durchgeführt wird, welcher die Sequenz SEQ ID NO: 34 aufweist;
- wobei in Schritt C), der Amplifikationsschritt einer PCR vom Nested-Typ umfassend zwei PCR-Runden ist und wobei die erste PCR-Runde mit einem Primersatz durchgeführt wird, welcher ein PCR-Nukleinsäure-Produkt mit einer Länge umfassend zwischen 200 bp und 300 bp ergibt, und wobei die zweite PCR-Runde mit einem Primersatz durchgeführt wird, welcher ein finales PCR-Nukleinsäureprodukt mit einer Länge umfassend zwischen 90 bp und 160 bp ergibt.

7. Verfahren nach Anspruch 6, wobei in Schritt C), der PCR-Amplifikationsschritt eine Nested-PCR umfassend zwei PCR-Runden ist,

- wobei, für Maus, Ratte oder Mensch, die erste PCR-Runde durchgeführt wird mit dem Primersatz:

Vorwärtsprimer 5'-TGTCCCTAGCCATTGCTGATATGC-3', und
Rückwärtsprimer 5'-GCAATCTTCATGATGGCCTTAGTC-3'; und

- wobei für Mensch, die zweite PCR-Runde durchgeführt wird mit den folgenden Primersätzen:

Vorwärtsprimer FAM-ATGTGCTATTTTCAACAGCGTCCATC-3', und
Rückwärtsprimer VIC-GCAATCTTCATGATGGCCTTA-3';
und

- wobei für Maus und Ratte, die zweite PCR-Runde durchgeführt wird mit den folgenden Primersätzen:

Vorwärtsprimer 5'-TTTGTGCCCCCGTCTGGAT-3', und
Rückwärtsprimer 5'-GCCTTAGTCCGCGAATTG-3'.

8. Verfahren nach Anspruch 7, wobei die Editingrate für jede editierte und uneditierte Form oder das Profil der editierten Formen der 5HTR2C-mRNA mit einem SSCP-Verfahren bestimmt wird, welches in der Lage ist, das Editingprofil für jede der editierten und uneditierten separaten Formen der mRNA bereitzustellen, wobei das SSCP-Verfahren **dadurch gekennzeichnet ist, dass** es nach den Schritten A), B) und C) die folgenden Schritte umfasst:

D) falls geeignet, Aufreinigung der in Schritt C) erhaltenen PCR-Produkte;
E) falls geeignet, Quantifizierung der in Schritt D) erhaltenen PCR-Produkte;
F) Dissoziation der doppelsträngigen cDNAs in einzelsträngige cDNAs, insbesondere durch ein Erwärmen gefolgt von einem plötzlichen Abkühlen;
G) Trennung der einzensträngigen cDNAs durch Kapillarelektrophorese; und
H) Erhalten des Editingprofils durch Bestimmen der Fluoreszenz und, falls geeignet, Erfassen der Profildaten mittels des Auswertungssystems, welches mit dem Fluoreszenzreader verbunden ist.

9. Verfahren nach einem der Ansprüche 1 bis 8, wobei die ADAR-Expressionsprodukte ADAR1-, Isoformen 150 und/oder 110, und die ADAR2-Genexpressionsprodukte sind, bevorzugt die Expressionsprodukte des humanen Gens, welches das ADAR1-, die Isoformen 150-kD und/oder 110-kD-Protein, und das ADAR2-Protein kodiert.

10. Verfahren nach einem der Ansprüche 1 bis 9, wobei die ADAR-Expressionsprodukte die ADAR-mRNAs sind.

11. Verfahren nach Anspruch 10, wobei die Bestimmung der ADAR-mRNA mit einem Verfahren durchgeführt wird, welches die folgenden Schritte umfasst:

A) Extrahieren der Gesamt-RNA der Hautzelllinie Probenzellen, gefolgt von, falls geeignet, einer Aufreinigung der mRNAs;
B) reverse Transkription der in Schritt A) extrahierten RNAs; und
C) PCR-Amplifikation der in Schritt B) erhaltenen cDNAs unter Verwendung wenigstens eines Primerpaars, das für jede ADAR-mRNA spezifisch ist, die quantifiziert und/oder qualitativ analysiert werden soll, und wobei in Schritt C) die Primerpaare, die für die ADAR-mRNA-PCR-Amplifikation spezifisch sind, ausgewählt sind aus der Gruppe bestehend aus:

- für die Amplifikation von mRNA von humanen ADAR1-150-Isoformen:

Vorwärts: 5'-GCCTCGCGGGCGCAATGAATCC-3',
Rückwärts: 5'-CTTGCCCTTCTTTGCCAGGGAG-3';

- für die Amplifikation von mRNA von humanen ADAR1-110-Isoformen:

Vorwärts: 5'-CGAGCCATCATGGAGATGCCCTCC-3',
Rückwärts: 5'-CATAGCTGCATCCTGCTTGGCCAC-3';

- für die Amplifikation von mRNA von humanem ADAR2:

Vorwärts: 5'-GCTGCGCAGTCTGCCCTGGCCGC-3',
Rückwärts: 5'-GTCATGACGACTCCAGCCAGCAC-3';

- für die Amplifikation von mRNA von Mäuse-ADAR1-150-Isoformen:

Vorwärts: 5'-GTCTCAAGGGTTCAGGGGACCC-3',
Rückwärts: 5'-CTCCTCTAGGGAATTCCTGGATAC-3';

- für die Amplifikation von mRNA von Mäuse-ADAR1-110-Isoformen:

Vorwärts: 5'-TCACGAGTGGGCAGCGTCCGAGG-3',
Rückwärts: 5'-CTCCTCTAGGGAATTCCTGGATAC-3'; und

- für die Amplifikation von mRNA von Mäuse-ADAR2:

Vorwärts: 5'-GCTGCACAGTCTGCCTTGGCTAC-3',
Rückwärts: 5'-GCATAAAGAAACCTGAGCAGGGAC-3'.

12. Verfahren nach einem der Ansprüche 1 bis 9, wobei die ADAR-Expressionsprodukte die ADAR-Proteine sind.

13. Verwendung eines Verfahrens nach einem der Ansprüche 1 bis 12, zum Identifizieren *in vitro*, ob ein Patient eine Pathologie aufweist oder ein Risiko aufweist, eine Pathologie zu entwickeln, die mit einer Veränderung des Mechanismus des mRNA-Editing des 5HTR2C im Gehirn in Beziehung steht, in einer biologischen Probe des zu untersuchenden Patienten, die Hautzellen enthält, wobei das Verfahren die folgenden Schritte umfasst:

a) Bestimmen der Editingrate oder des -profils der editierten Formen der 5HTR2C-mRNA, die in der Hautzellprobe exprimiert wird; und, gegebenenfalls

b) Bestimmen der Art und/oder der Menge der in der Hautzellprobe exprimierten ADARs; und

c) Feststellen, ob der Patient eine solche Pathologie aufweist oder ein Risiko aufweist, eine solche Pathologie zu entwickeln, durch Vergleichen der Editingrate oder des -profils der 5HTR2C-mRNA und der Art und/oder der Menge der in der Probe exprimierten ADARs, die Schritt a) und b) erhalten wurden, mit einer charakteristischen Kontrolleditingrate oder einem charakteristischen Kontrollprofil der 5HTR2C-mRNA und einem exprimierten ADAR-Profil, welche von normalen Patienten oder von Patienten erhalten wurden, die Pathologien aufweisen, die mit einer Veränderung des Mechanismus dieses mRNA-Editing im Gehirn in Beziehung stehen.

14. Verwendung eines Verfahrens nach einem der Ansprüche 1 bis 12, zum Bestimmen *in vitro*, ob eine Pathologie, die ein Patient aufweist, mit einer Veränderung des Mechanismus des mRNA-Editing des 5HTR2C im Gehirn aufweist, in einer biologischen Probe des zu untersuchenden Patienten, die Hautzellen enthält, wobei das Verfahren die folgenden Schritte umfasst:

a) Bestimmen der Editingrate oder des -profils der editierten Formen der 5HTR2C-mRNA, die in der Hautzellprobe exprimiert wird; und gegebenenfalls

b) Bestimmen der Art und/oder der Menge der in der Hautzellprobe exprimierten ADARs; und

c) Feststellen, ob der Patient eine solche Pathologie aufweist oder ein Risiko aufweist, eine solche Pathologie zu entwickeln, durch Vergleichen der Editingrate oder des -profils der 5HTR2C-mRNA und der Art und/oder der Menge der in der Probe exprimierten ADARs, die Schritt a) und b) erhalten wurden, mit einer charakteristischen Kontrolleditingrate oder einem charakteristischen Kontrollprofil der 5HTR2C-mRNA und einem exprimierten ADAR-Profil, welche von normalen Patienten oder von Patienten erhalten wurden, die Pathologien aufweisen, die nicht mit einer Veränderung des Mechanismus dieses mRNA-Editing in Beziehung stehen.

15. Verwendung eines Verfahrens nach einem der Ansprüche 1 bis 12, zum Identifizieren *in vitro* eines Mittels, das *in vivo* das Editing der 5HTR2C-mRNA im Gehirn eines Säugers moduliert, in einer biologischen Probe des Säugers enthaltend Hautzellen, wobei der Kandidatenmodulator des 5HTR2C-mRNA-Editing dem Säuger vorher verabreicht wird, wobei das Verfahren die folgenden Schritte umfasst:

a) Bestimmen der Wirkungen des Modulators

- auf die Editingrate oder das -profil der editierten Formen der 5HTR2C-mRNA, die in der Hautzellprobe exprimiert wird; und gegebenenfalls
- auf die Art und/oder die Menge der in der Hauzellprobe exprimierten ADARs durch Vergleichen der Editingrate oder des -profils der 5HTR2C-mRNA und der Art und/oder der Menge der in der Probe exprimierten ADARs, die in Schritt a) erhalten wurden, mit der Editingrate oder dem -profil der 5HTR2C und der Art und/oder Menge der ADARs, die von Hautzellkontrollproben erhalten wurden.

16. Verwendung eines Verfahren nach einem der Ansprüche 1 bis 12, zum Bestimmen *in vivo* der Wirksamkeit einer Wirkstoffs, der zur Vorbeugung und oder Behandlung in einem Patienten einer Pathologie verwendet wird, welche mit dem Mechanismus des mRNA-Editing des 5HTR2C im Gehirn in Beziehung steht, in einer biologischen Probe des Patienten enthaltend Hautzellen, die vor und während und/oder nach der Behandlung erhalten wurde, wobei das Verfahren die folgenden Schritte umfasst:

a) Bestimmen in den Proben enthaltend Hautzellen, die vor und während und/oder nach der Behandlung erhalten wurden:

- der Editingrate oder des -profils der editierten Formen der 5HTR2C-mRNA, die in den Hautzellproben exprimiert wurde; und gegebenenfalls
- der Art und/oder der Menge der ADARs, die in den Hautzellproben exprimiert wurden; und

b) Bestimmen der Wirksamkeit des Wirkstoffs durch ein Vergleichen der Editingrate oder des -profils der 5HTR2C-mRNA und der Natur und/oder dem Menge der ADARs, die in den Schritt a) erhaltenen Proben exprimiert wurden, wobei eine Modulation der Editingrate oder des -profils und der Art und/oder dem Menge der exprimierten ADARs, die eine Editingrate oder ein -profil oder eine Art und/oder eine Menge der exprimierten ADARs ergeben, die annähernd gleich oder gleich zu dem sind, was für normale Patienten beobachtet wird, eine wirksame Behandlung anzeigt.

17. Verwendung eines Verfahrens nach einem der Ansprüche 1 bis 12, zum Bestimmen, ob ein Patient auf eine Behandlung anspricht oder nicht anspricht auf eine Behandlung einer Pathologie, die aus einer Veränderung des Mechanismus des mRNA-Editing des 5HTR2C im Gehirn resultiert oder dadurch hervorgerufen wird, ferner umfassend die Schritte:

c) Bestimmen, ob der Patient auf die Behandlung anspricht oder nicht anspricht durch ein Bestimmen der Veränderung der Editingrate oder des -profils und, gegebenenfalls, der Art und/oder der Menge der ADARs, die nach einem Behandlungszeitraum exprimiert werden (d.h. 15 Tage, 30 Tage, 2 Monate, 6 Monate, usw.), durch ein Vergleichen mit der Editingrate oder dem -profil und, gegebenenfalls, der Art und/oder der Menge der ADARs, die vor dem Beginn der Behandlung exprimiert wurden.

18. Verwendung eines Verfahrens nach einem der Ansprüche 1 bis 12, zum Identifizieren eines Mittels, welches *in vitro* das Editing der 5HTR2C-mRNA in einem Säuger moduliert, wobei das Verfahren die folgenden Schritte umfasst:

a) Erhalten einer biologischen Probe enthaltend Zellen von einer Säugerhautzelllinie, gegebenenfalls, wobei die Zellen rekombinante Zellen sein können;

b) Kontaktieren der biologischen Probe in der Anwesenheit eines Kandidatenmodulators des 5HTR2C-mRNA-Editings; und, gegebenenfalls

c) Bestimmen der Wirkungen des Modulators

- auf die Editingrate oder das -profil der editierten Formen der 5HTR2C-mRNA, die in der Hautzellprobe exprimiert werden; und,

- auf die Art und/oder die Menge der ADARs, die in der Hautzellprobe exprimiert wurden, durch ein Vergleichen der Editingrate oder des -profils der 5HTR2C-mRNA und der Art und/oder der Menge der ADARs, die in der in Schritt c) erhaltenen Probe exprimiert wurden, mit der Editingrate oder dem -profil der 5HTR2C-mRNA und der Art und/oder der Menge der ADARs, die von einer Kontrollprobe von Hautzellen erhalten wurden.

**Revendications**

1. Procédé mettant en oeuvre un échantillon biologique constitué d'un tissu périphérique contenant des cellules pour évaluer l'altération pathologique de l'édition A en I ADAR dépendante de l'ARNm du 5HTR2C dans le cerveau, dans lequel ledit tissu périphérique contenant des cellules est un échantillon de peau de mammifère choisi parmi le groupe consistant en une lignée cellulaire de peau, de cellules cultivées dérivées de la peau et un échantillon de tissu de peau, et dans lequel l'altération pathologique de l'édition A en I ADAR dépendante de l'ARNm du 5HTR2C dans le cerveau est détectée en mesurant l'édition du 5HTR2C dans l'échantillon de peau.

2. Procédé selon la revendication 1, dans lequel ladite pathologie est choisie parmi le groupe constitué des troubles mentaux, la schizophrénie, la dépression, le suicide lié à la dépression ou un comportement alimentaire anormal.

3. Procédé selon la revendication 1 ou 2, dans lequel ce procédé comprend en outre une étape de détermination des produits d'expression des ADARs contenus dans un deuxième échantillon de tissu périphérique consistant en un échantillon de sang contenant des cellules dudit patient ou mammifère.

4. Procédé selon la revendication 3, dans lequel les cellules exprimant des ADARs dans l'échantillon de sang sont des globules blancs ou des leucocytes.

5. Procédé selon l'une quelconque des revendications 1 à 4, dans lequel ledit taux d'édition ou ledit profil des formes éditées est déterminé pour toutes les formes éditées et non-éditées dudit ARNm de 5HTR2C.

6. Procédé selon l'une quelconque des revendications 1 à 5, dans lequel le taux d'édition de chaque forme éditée et non éditée ou le profil des formes éditées dudit ARNm de 5HTR2C est déterminée par un procédé comprenant les étapes suivantes :

A) extraction des ARN totaux de ladite lignée cellulaire de peau, desdites cellules cultivées dérivées de la peau ou dudit tissu de la peau, suivie, le cas échéant, d'une purification des ARNm ;

B) transcription inverse de l'ARN extrait dans l'étape A) ; et

C) amplification par PCR des ADNc obtenus dans l'étape B) en utilisant au moins une paire d'amorces spécifiques pour le fragment d'ARNm de 5HTR2C contenant les sites d'édition pouvant être édités, ladite paire d'amorces étant choisie de manière à pouvoir amplifier toutes les formes éditées et les formes non éditées potentiellement présentes dans l'extrait d'ARN,

et

- dans laquelle l'étape B) de transcription inverse est mise en oeuvre en utilisant une amorce oligonucléotidique spécifique du gène 5HTR2C ayant la séquence SEQ ID No. 34 ;

- dans laquelle dans l'étape C), l'étape d'amplification par PCR est une PCR de type nichée comprenant deux cycles de PCR, dans laquelle le premier cycle de PCR est mis en oeuvre par un jeu d'amorces résultant en un produit d'acide nucléique de PCR ayant une longueur comprise entre 200 pb et 300 pb, et dans laquelle le second cycle de PCR est mis en oeuvre par un jeu d'amorces résultant en un produit final d'acide nucléique de PCR ayant une longueur comprise entre 90 pb et 160 pb.

7. Procédé selon la revendication 6, dans lequel dans l'étape C), l'étape d'amplification par PCR est une PCR de type nichée comprenant deux cycles de PCR,

- dans laquelle, pour la souris, le rat ou l'homme, le premier cycle de PCR est mis en oeuvre par un jeu d'amorces :

Amorce sens 5'-TGTCCCTAGCCATTGCTGATATGC-3', et
Amorce anti-sens 5'-GCAATCTTCATGATGGCCTTAGTC-3' ; et

- dans laquelle pour l'homme, le second cycle de PCR est mis en oeuvre par le jeu d'amorces suivant :

Amorce sens FAM-ATGTGCTATTTTCAACAGCGTCCATC-3'
Amorce anti-sens VIC-GCAATCTTCATGATGGCCTTA-3' ;
et

- dans laquelle pour la souris et le rat, le second cycle de PCR est mis en oeuvre par le jeu d'amorces suivant :

Amorce sens 5'-TTTGTGCCCCGTCTGGAT-3', et
Amorce anti-sens 5'-GCCTTAGTCCGCGAATTG-3'.

8. Procédé selon la revendication 7, dans lequel le taux d'édition pour chaque forme éditée et non-éditée ou le profil des formes éditées dudit ARNm de 5HTR2C est déterminé par une technique SSCP capable de déterminer le profil d'édition pour chacune des formes distinctes éditées et non-éditées dudit ARNm, ladite technique SSCP étant **caractérisée en ce qu'**elle comprend après les étapes A), B) et C) les étapes suivantes :

D) le cas échéant, une purification des produits de PCR obtenus à l'étape C) ;
E) le cas échéant, une quantification des produits de PCR obtenus à l'étape D);
F) la dissociation des ADNc double brin en ADNc simple brin, en particulier par chauffage suivi d'un refroidissement brusque
G) la séparation des ADNc simple brin par électrophorèse capillaire ; et
H) l'obtention du profil d'édition par la lecture de la fluorescence et, le cas échéant, l'acquisition des données du profil au moyen du système d'exploitation associé au lecteur de fluorescence.

9. Procédé selon l'une quelconque des revendications 1 à 8, dans lequel les produits d'expression des ADARs sont les produits d'expression du gène de l'ADAR1, isoformes 150 et/ou 110, et du gène de l'ADAR2, de préférence les produits d'expression du gène humain codant pour la protéine ADAR1, isoformes 150-kD et/ou 110-kD, et pour la protéine ADAR2.

10. Procédé selon l'une quelconque des revendications 1 à 9, dans lequel les produits d'expression des ADARs sont les ARNm d'ADAR.

11. Procédé selon la revendication 10, dans lequel la détermination des ARNm d'ADAR est mis en oeuvre par un procédé qui comprend les étapes suivantes :

A) l'extraction des ARN totaux desdites cellules d'échantillon, suivie, le cas échéant, par la purification des ARNm ;
B) la transcription inverse des ARN extraits à l'étape A) ; et
C) l'amplification par PCR des ADNc obtenus à l'étape B) en utilisant au moins une paire d'amorces spécifique pour chacun des ARNm d'ADAR à quantifier et/ou à analyser qualitativement, et dans laquelle à l'étape C), la paire d'amorces spécifique pour l'amplification par PCR des ARNm d'ADAR est choisie parmi le groupe consistant en :

- pour l'amplification de l'ARNm de l'isoforme ADAR1-150 humain :

Sens : 5'-GCCTCGCGGGCGCAATGAATCC-3' ;
Anti-sens : 5'-CTTGCCCTTCTTTGCCAGGGAG-3' ;

- pour l'amplification de l'ARNm de l'isoforme ADAR1-110 humain :

Sens : 5'-CGAGCCATCATGGAGATGCCCTCC -3' ;
Anti-sens : 5'-CATAGCTGCATCCTGCTTGGCCAC-3' ;

- pour l'amplification de l'ARNm d'ADAR2 humain :

Sens : 5'-GCTGCGCAGTCTGCCCTGGCCGC-3' ;
Anti-sens : 5'-GTCATGACGACTCCAGCCAGCAC -3' ;

- pour l'amplification de l'ARNm de l'isoforme ADAR1-150 murin :

Sens : 5'-GTCTCAAGGGTTCAGGGGACCC-3' ;
Anti-sens : 5'-CTCCTCTAGGGAATTCCTGGATAC-3' ;

- pour l'amplification de l'ARNm de l'isoforme ADAR1-110 murin :

Sens : 5'-TCACGAGTGGGCAGCGTCCGAGG-3' ;
Anti-sens : 5'-CTCCTCTAGGGAATTCCTGGATAC-3' ; et

- pour l'amplification de l'ARNm d'ADAR2 murin :

Sens : 5'-GCTGCACAGTCTGCCTTGGCTAC-3' ;
Anti-sens : 5'-GCATAAAGAAACCTGAGCAGGGAC-3'.

12. Procédé selon l'une quelconque des revendications 1 à 9, dans lequel les produits d'expression des ADARs sont les protéines ADAR.

13. Utilisation d'un procédé selon l'une quelconque des revendications 1 à 12, pour identifier *in vitro* si un patient présente une pathologie ou est à risque de développer une pathologie liée à une altération du mécanisme de l'édition de l'ARNm du 5HTR2C dans le cerveau, à partir d'un échantillon biologique du patient à tester contenant des cellules de peau, dans lequel ce procédé comprend les étapes suivantes de :

a) détermination du taux d'édition ou du profil des formes éditées dudit ARNm du 5HTR2C exprimé dans ledit échantillon de cellules de peau ; et, le cas échéant
b) détermination du type ou/et de la quantité d'ADAR exprimé dans ledit échantillon de cellules de peau ; et
c) identification si ledit patient présente ou est à risque de développer une telle pathologie par comparaison du taux ou profil d'édition dudit ARNm du 5HTR2C et le type ou/et la quantité d'ADAR exprimé dans ledit échantillon obtenu à l'étape a) et b) avec le taux ou profil d'édition témoin caractéristique de l'ARNm du 5HTR2C et du profil des ADARs exprimés obtenu pour des patients normaux ou pour des patients présentant des pathologies liées à une altération du mécanisme de l'édition de cet ARNm dans le cerveau.

14. Utilisation d'un procédé selon l'une quelconque des revendications 1 à 12, pour déterminer *in vitro* si une pathologie présentée par un patient est liée à une altération du mécanisme de l'édition de l'ARNm du 5HTR2C dans le cerveau, à partir d'un échantillon biologique du patient à tester contenant des cellules de peau, dans lequel ce procédé comprend les étapes suivantes de :

a) détermination du taux d'édition ou du profil des formes éditées dudit ARNm du 5HTR2C exprimé dans ledit échantillon de cellules de peau ; et, le cas échéant
b) détermination du type ou/et de la quantité d'ADARs exprimé dans ledit échantillon de cellules de peau ; et
c) identification si ledit patient présente ou est à risque de développer une telle pathologie par comparaison du taux ou profil d'édition dudit ARNm du 5HTR2C et le type ou/et la quantité d'ADARs exprimé dans ledit échantillon obtenu à l'étape a) et b) avec le taux ou profil d'édition témoin caractéristique de l'ARNm de 5HTR2C et le profil d'ADARs exprimés obtenu pour les patients normaux ou pour les patients présentant des pathologies connues pour ne pas être liées à une altération du mécanisme de l'édition de cet ARNm.

15. Utilisation d'un procédé selon l'une quelconque des revendications 1 à 12, pour identifier *in vitro* un agent modulant *in vivo* l'édition de l'ARNm du 5HTR2C dans le cerveau d'un mammifère, à partir d'un échantillon biologique dudit mammifère contenant des cellules de peau, ledit candidat modulateur de l'édition de l'ARNm du 5HTR2C étant préalablement administré audit mammifère, ledit procédé comprenant les étapes suivantes de :

a) détermination des effets dudit modulateur

EP 2 162 550 B1

- sur le taux d'édition ou le profil des formes éditées dudit ARNm du 5HTR2C exprimé dans ledit échantillon de cellules de peau ; et, le cas échéant

- sur le type ou/et la quantité des ADAR exprimés dans ledit échantillon de cellules de peau, par comparaison du taux ou profil d'édition dudit ARNm du 5HTR2C et le type ou/et la quantité des ADARs exprimés dans l'échantillon obtenu à l'étape a) avec le taux ou profil d'édition dudit ARNm du 5HTR2C et le type ou/et la quantité d'ADARs exprimés obtenu à partir de l'échantillon témoin de cellule de peau.

16. Utilisation d'un procédé selon l'une quelconque des revendications 1 à 12, pour déterminer *in vitro* l'efficacité d'un médicament utilisé pour la prévention ou pour le traitement chez un patient d'une pathologie liée à une altération du mécanisme d'édition de l'ARNm de 5HTR2C dans le cerveau, à partir d'un échantillon biologique du patient contenant des cellules de peau obtenu avant et pendant ou/et après le traitement, dans lequel ce procédé comprend les étapes suivantes de :

a) détermination dans ledit échantillon contenant des cellules de peau obtenu avant et pendant ou/et après le traitement :

- le taux ou profil d'édition des formes éditées dudit ARNm du 5HTR2C exprimé dans lesdits échantillons de cellules de peau ; et, le cas échéant

- le type ou/et la quantité des ADAsR exprimés dans lesdits échantillons de cellules de peau ; et

b) détermination de l'efficacité dudit médicament en comparant le taux ou profil d'édition dudit ARNm du 5HTR2C et le type ou/et quantité des ADARs exprimés dans lesdits échantillons obtenus à l'étape a), la modulation du taux ou profil d'édition et le type ou/et la quantité des ADARs exprimés résultant en un taux ou profil d'édition et un type et/ou une quantité d'ADARs exprimés proche ou égale de celle observée chez les patients normaux étant significatif de l'efficacité du traitement.

17. Utilisation d'un procédé selon l'une quelconque des revendications 1 à 12, pour déterminer si un patient répond ou ne répond pas à un traitement d'une pathologie résultant ou provoqué par la modification du mécanisme d'édition de l'ARNm de 5HTR2C dans le cerveau, comprenant en outre les étapes de:

c) détermination si le patient répond ou ne répond pas au traitement en observant la modification du taux ou profil d'édition et, le cas échéant, le type ou/et la quantité d'ADARs exprimés après une période de traitement (i.e. 15 jours, 30 jours, 2 mois, 6 mois, etc.) en comparant avec le taux ou profil d'édition et, le cas échéant, le type ou/et la quantité d'ADARs exprimés avant le début du traitement.

18. Utilisation d'un procédé selon l'une quelconque des revendications 1 à 12, pour identifier *in vitro* un agent qui module *in vitro* l'édition de l'ARNm du 5HTR2C chez un mammifère, ledit procédé comprenant les étapes suivantes de:

a) obtention d'un échantillon biologique contenant des cellules d'une lignée cellulaire de peau de mammifère, le cas échéant, lesdites cellules peuvent être des cellules recombinantes ;

b) mise en contact dudit échantillon biologique en présence d'un candidat modulateur de ladite édition de l'ARNm du 5HTR2C ; et, le cas échéant

c) détermination des effets dudit modulateur

- sur le taux ou profil d'édition des formes éditées dudit ARNm du 5HTR2C exprimé dans ledit échantillon de cellules de peau ; et

- sur le type ou/et la quantité d'ADARs exprimés dans ledit échantillon de cellules de peau, par comparaison du taux ou profil d'édition dudit ARNm du 5HTR2C et le type ou/et la quantité d'ADARs exprimés dans ledit échantillon obtenu à l'étape c) avec le taux ou profil d'édition dudit ARNm du 5HTR2C et le type ou/et la quantité d'ADARs exprimés obtenu à partir d'un échantillon témoin de cellules de peau.

**FIGURE 1**

**FIGURE 2A**

**FIGURE 2B**

**FIGURE 3A**

**FIGURE 3B**

**FIGURE 4A**

**FIGURE 4B**

**FIGURE 5A**

**FIGURE 5B**

FIGURE6A     FIGURE 6B     FIGURE 6C

## REFERENCES CITED IN THE DESCRIPTION

### Non-patent literature cited in the description

- **LERER et al.** *Mol. Psychiatry,* 2001, vol. 6, 579-585
- **TECOTT et al.** *Nature,* 1995, vol. 374, 542-546
- **CHOU-GREEN et al.** *Physiol. Behav.,* 2003, vol. 79, 217-226
- **IWAMOTO et al.** *Mol. Psychiatry,* 2004, vol. 9, 406-416
- **CASTENSSON et al.** *Biol. Psychiatry,* 2003, vol. 54, 1212-1221
- **SEEBURG.** *Neuron,* 2002, vol. 35, 17-20
- **PRICE et al.** *J. Biol. Chem.,* 2001, vol. 276, 44663-44668
- **ENGLANDER et al.** *J. Neurosci.,* 2005, vol. 25, 648-651
- **NISWENDER et al.** *Neuropsychopharmacology,* 2001, vol. 24, 478-491
- **SODHI et al.** *Mol. Psychiatry,* 2001, vol. 6, 373-379
- **GUREVICH et al.** *Neuron,* 2002, vol. 34, 349-356
- **TOHDA et al.** *J. Pharmacol. Sci.,* 2006, vol. 100, 427-432
- **TOHDA et al.** *Neurosci. Res.,* 1996, vol. 24, 189-193
- **PASQUALETTI et al.** *Neuroscience,* 1999, vol. 92, 601-611
- **MARAZZITI et al.** *Neuropsychobiology,* 2001, vol. 43, 123-126
- **SLOMINSKI et al.** *FASEB J.,* 2002, vol. 16, 896-898
- **SLOMINSKI et al.** *J. Cell. Phys.,* 2003, vol. 196, 144-153
- **MAAS S et al.** *J. Biol. Chem,* 1996, vol. 271, 12221-26
- **SERGEEVA OA et al.** *Cell Mol Neurobiol.,* 2007, vol. 27, 669-680
- **ORLOWSKI RJ et al.** 8A1 gene transcripts of systemic lupus erythematosus T lymphocytes. *Immunology,* 2008
- **WANG P et al.** phosphodiesterase 8A (PDEA8) splice variants: cloning, gene organization and tissue distribution. *Gene,* 2001, vol. 280, 183-194
- **YANG W et al.** *Brain Res Mol Brain Res,* 2004, vol. 124 (1), 70-78
- **DRACHEVA et al.** *Molecular Psychiatry,* 2007, 1-10
- **YANG et al.** *Beyond the Identification of Transcribed Sequences: Functional, Evolutionary and Expression Analysis, 12th International Workshop,* 25 October 2002
- **SLOMINSKI et al.** *J. Cell Phy.,* 2003, vol. 196, 144-153
- **FITZGERALD et al.** *Neuropsychopharmacology,* 1999, vol. 21 (2S), 82S-90S